(19) Europäisches Patentamt
European Patent Office
Office européen des brevets

(11) Publication number: 0 475 238 A2

(12) **EUROPEAN PATENT APPLICATION**

(21) Application number: **91114803.9**

(22) Date of filing: **03.09.91**

(51) Int. Cl.[5]: **C07D 303/16**, C08G 59/02, C08G 59/20

(30) Priority: **13.09.90 US 582048**

(43) Date of publication of application: **18.03.92 Bulletin 92/12**

(84) Designated Contracting States: **AT BE CH DE DK ES FR GB IT LI NL SE**

(71) Applicant: **THE DOW CHEMICAL COMPANY**
**2030 Dow Center Abbott Road P.O. Box 1967**
**Midland Michigan 48640-1967(US)**

(72) Inventor: **Hefner, Robert E., Jr.**
**109 Wedgewood**
**Lake Jackson, Texas 77566(US)**
Inventor: **Earls, Jimmy D.**
**209 Banyan**
**Lake Jackson, Texas 77566(US)**

(74) Representative: **Sternagel, Hans-Günther, Dr. et al**
**Patentanwalte Dr. Michael Hann, Dr. H.-G. Sternagel, Dr. H. Dörries, Sander Aue 30**
**W-5060 Bergisch Gladbach 2(DE)**

(54) **Mesogenic glycidyl esters.**

(57) Glycidyl esters are disclosed which contain mesogenic moieties which result in improved properties when cured.

EP 0 475 238 A2

The present invention concerns glycidyl esters of mono and polycarboxylic acids containing one or more mesogenic moieties, curable compositions and cured compositions thereof.

**BACKGROUND OF THE INVENTION**

Glycidyl esters of polycarboxylic acids are a specialized class of thermosettable resins with utility in a myriad of applications, notably coatings, adhesives, encapsulants, moldings, laminates, castings, electrical insulation, weatherable coatings, sealants, impregnants, plasticizers, fibers, or foams. The art describes numerous incremental improvements in the physical, mechanical, thermal and/or chemical resistant properties possessed by certain polyglycidyl esters relative to their polyglycidyl ether counterparts. This nonwithstanding, substantial room for improvement in one or more of the aforesaid properties of polyglycidyl esters is desirable for each of the aforementioned applications.

The present invention provides a method for improving the properties of mono and polyglycidyl esters as well as the curable and cured compositions thereof by incorporating one or more mesogenic moieties into the backbone chain of said glycidyl esters. These glycidyl esters exhibit ordering of the molecular chains in the melt phase and/or in the advanced compositions thereof. This morphology is susceptible to orientation during processing which can result in enhanced unidirectional mechanical properties. This is not possible to any significant extent with the conventional (non-mesogenic) glycidyl esters. The mesogenic structures incorporated into the backbone chain of the glycidyl esters and the polymer chains of the resultant polymers thereof are believed to be responsible for the improvement in properties.

One aspect of the present invention pertains to polyglycidyl esters containing one or more mesogenic moieties represented by the following Formula I

Formula I

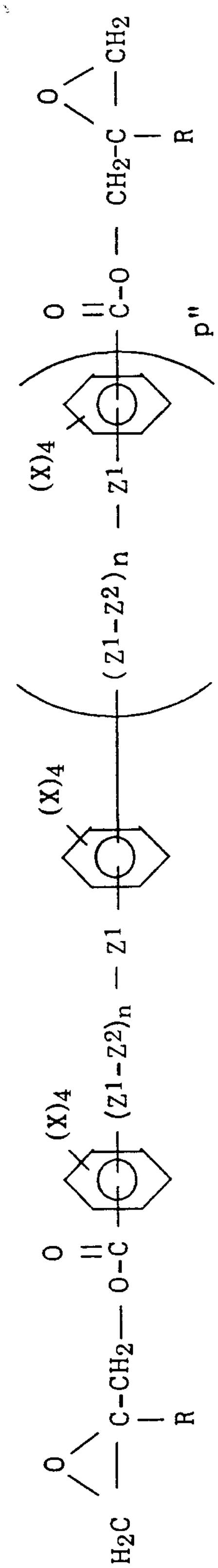

wherein at least 80 percent of the $-(Z^1-Z^2)n-Z^1-$linkages and the glycidyl ester groups are in the para position with respect to each other; each R and $R^1$ is independently hydrogen or an aliphatic hydrocarbon group having from 1 to 4 carbon atoms; each X is independently hydrogen, a hydrocarbyl or hydrocarbyloxy group having suitably from 1 to 12, more suitably from 1 to 6, most suitably from 1 to 4, carbon atoms, a halogen atom (preferably chlorine or bromine), $-NO_2$, or $-C\equiv N$; each $Z^1$ is independently a direct single bond, $-CR^1=CR^1-$,

$-CR^1=CR^1-CR^1=CR^1-$,
$-CR^1=N-N=CR^1-$, $-CR^1=CR^1-CO-O-(CHR^1)_{p'}-$,
$-CR^1=CR^1-O-CO-(CHR^1)_{p'}-$, $-(CHR^1)_{p'}-O-CO-CR^1=CR^1-$,
$-(CHR^1)_{p'}-CO-O-CR^1=CR^1-$, $-CR^1=CR^1-CO-O-$, $-O-CO-CR^1=CR^1-$,
$-CO-NR^1-$, $-NR^1-CO-$, $-CO-NR^1-NR^1-CO-$, $-C{\equiv}C-$, $-C{\equiv}C-C{\equiv}C-$,
$-CO-S-$, $-S-CO-$, $-CR^1=N-$, $-N=CR^1-$, $-O-CO-$, $-CO-O-$,
$-CR^1=CR^1-CO-$, $-CO-CR^1=CR^1-$, $-CR^1=CR^1-O-CO-$,
$-CO-O-CR^1=CR^1-$, $-CH_2-CH_2-CO-O-$, $-O-CO-CH_2-CH_2-$, $-N=N-$,

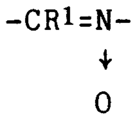 , 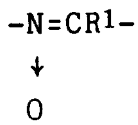 , 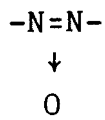 , 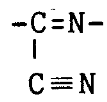 , 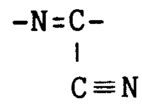

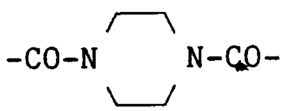 , 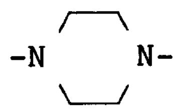 , 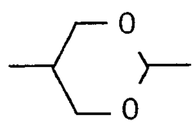 ,

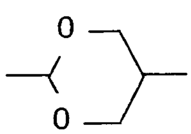 , 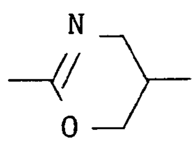 , 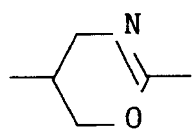 ,

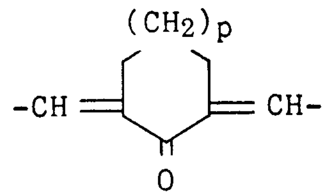 (p=0, 1, 2) ,

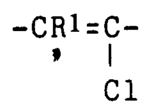 , 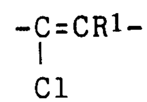 , 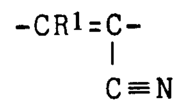 , 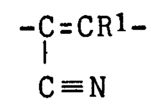 ,

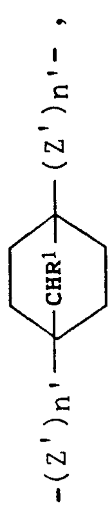 ,

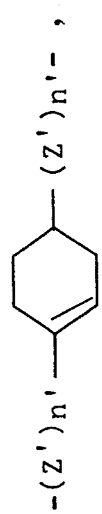 ,

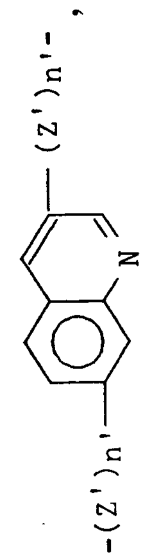 ,

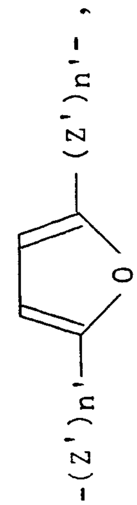 ,

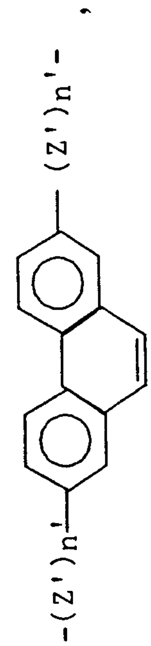 ,

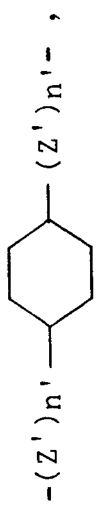 ,

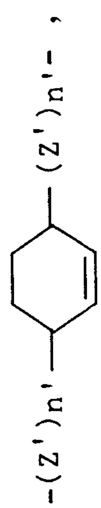 ,

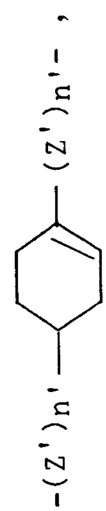 ,

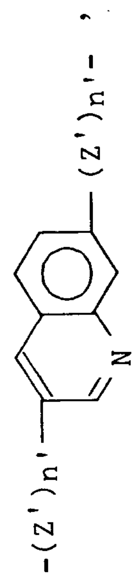 ,

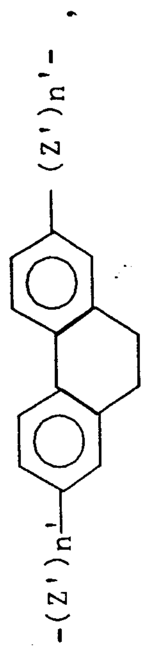 ,

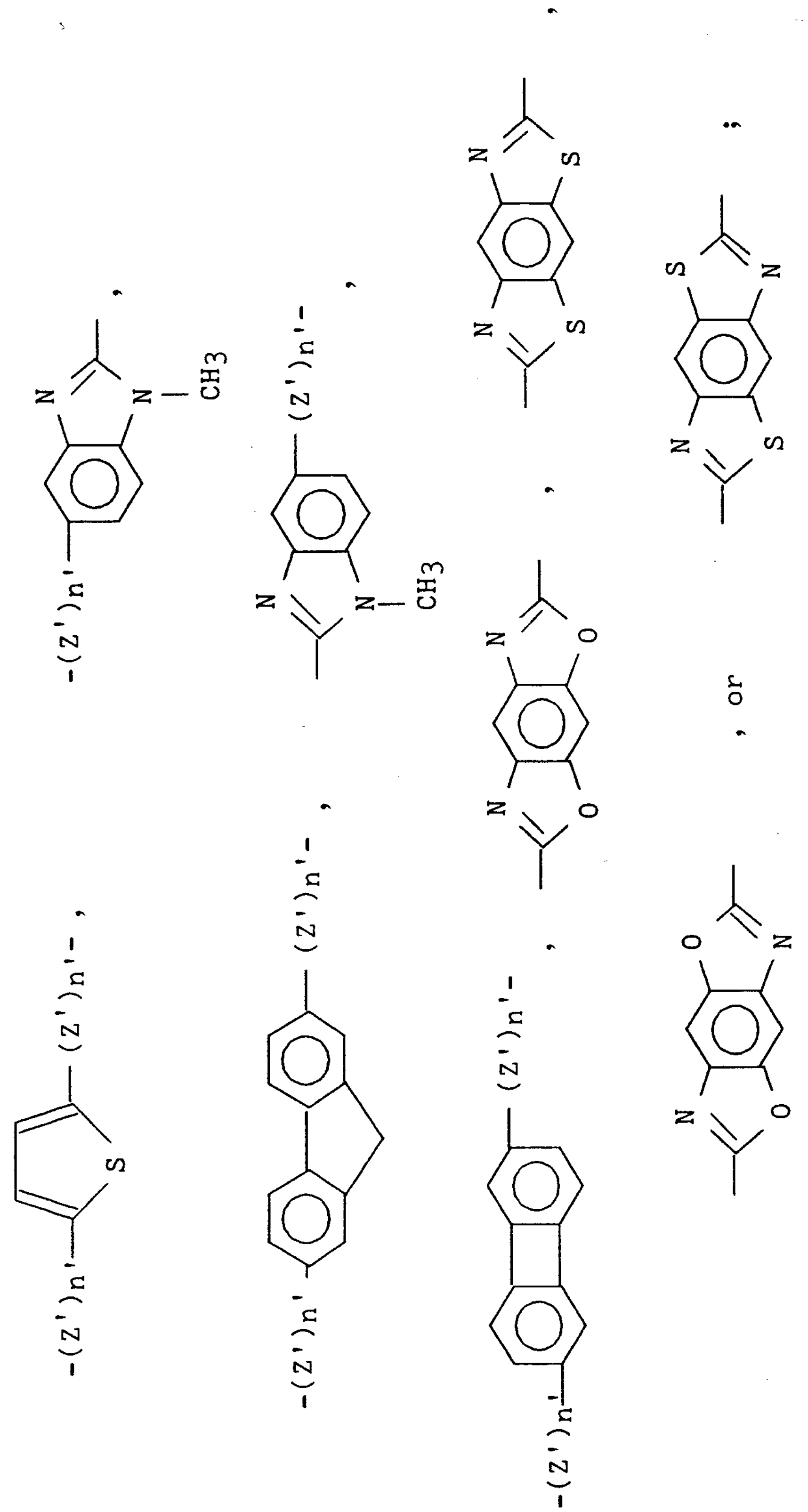

, or

$Z^2$ is a group represented by a cyclic or bicyclic ring system containing from 5 to 12 carbon atoms and may be cycloaliphatic, polycycloaliphatic, aromatic or a combination thereof; n is 0 to 2; p' is 1 or 2; p'' has a value of zero to 100; each Z' is independently a -CO-, -O-CO-, -CO-O-, -CO-$NR^1$-, or -$NR^1$-CO- group and each n' independently has a value of zero or one; with the proviso that the polyglycidyl ester of Formula I is not the polyglycidyl ester of 4,4'-dicarboxystilbene (R is H, X is H, n = O, p'' = O, $Z^1$ is -$CR^1$ = $CR^1$- wherein both $R^1$ groups are H) or the polyglycidyl ester or polymethylglycidyl ester of bis(4'-carboxyphenyl)-1,4-benzenediimine (R is H or $CH_3$, X is H, n = 1, p'' = O, $Z^1$ is $-CR^1=N-$ wherein $R^1$ is H and $Z^2$ is

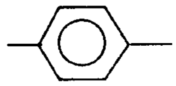 ).

Another aspect of the present invention pertains to polyglycidyl esters containing one or more mesogenic moieties represented by the following Formula II

Formula II

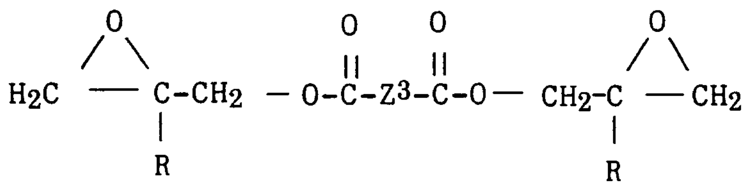

wherein $Z^3$ is

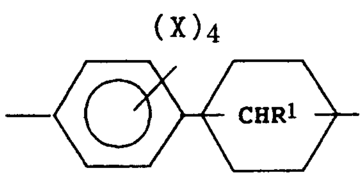 ,

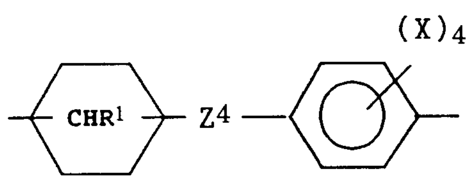 ,

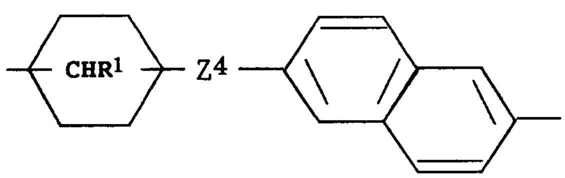 ,

or

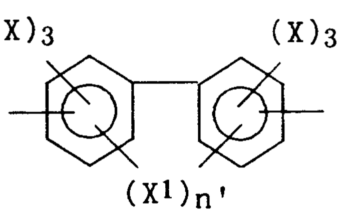 ;

and $Z^4$ is -CO-O-, -O-CO-, $-NR^1-CO-$ or $-CO-NR^1-$; $X^1$ is a hydrocarbyl group having from 1 to 10, preferably from 1 to 4, carbon atoms which can contain one or more heteroatoms selected from N, O, or S and may be saturated or unsaturated; each R and $R^1$ is independently hydrogen or an aliphatic hydrocarbon group having from 1 to 4 carbon atoms; each X is independently hydrogen, a hydrocarbyl or hydrocarbyloxy group having suitably from 1 to 12, more suitably from 1 to 6, most suitably from 1 to 4, carbon atoms, a halogen atom (preferably chlorine or bromine), $-NO_2$, or -C≡N; and each n' is independently zero or one.

Another aspect of the present invention pertains to monoglycidyl ester compounds containing one or more mesogenic moieties represented by the following Formula III

Formula III

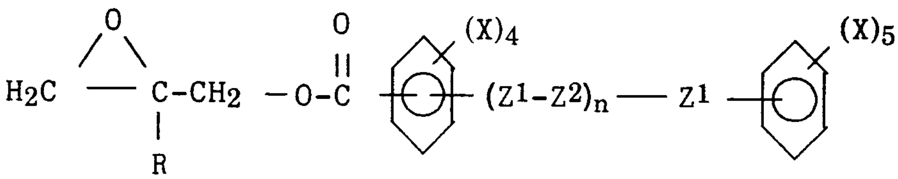

wherein at least 80 percent of the $-(Z^1-Z^2)_n-Z^1-$linkages and the glycidyl ester groups are in the para position with respect to each other; R, $R^1$, X, $Z^1$, $Z^2$, Z', n, p' and n' are as hereinbefore defined; with the proviso that the monoglycidyl ester of Formula III is not the monoglycidyl ester of 4-carboxybiphenyl (R = H, X = H, n = O, $Z^1$ = a direct single bond).

Another aspect of the present invention pertains to monoglycidyl ester compounds containing one or more mesogenic moieties represented by the following Formula IV

Formula IV

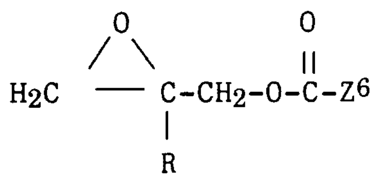

wherein R, $R^1$, X, $X^1$, $Z^3$, $Z^4$ and n' are as hereinbefore defined and $Z^6$ is

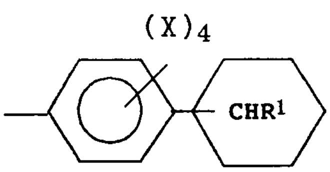 , 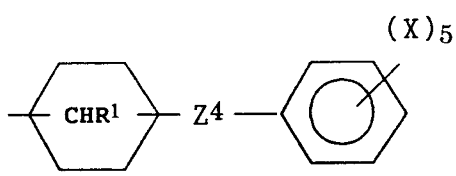 ,

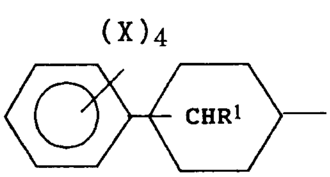 , 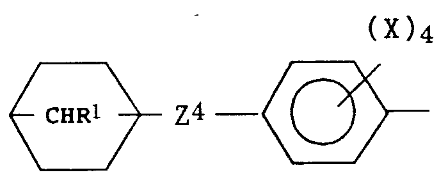 ,

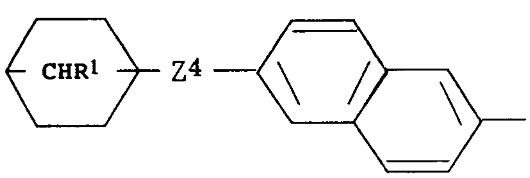 ,

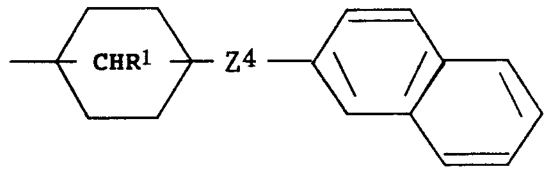

,

or

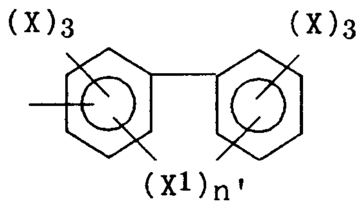

;

Another aspect of the present invention pertains to advanced polyglycidyl ester compositions prepared by reacting

(A) one or more of the polyglycidyl esters containing one or more mesogenic moieties, said polyglycidyl esters being those represented by either Formula I, with the proviso that the polyglycidyl ester of Formula I may include the polyglycidyl ester of 4,4'-dicarboxystilbene or the polyglycidyl ester or polymethylglycidyl ester of bis(4'-carboxyphenyl)-1,4-benzendiimine; or Formula II; with

(B) at least one compound having an average of more than one active hydrogen atom per molecule; and wherein components (A) and (B) are employed in quantities which provide a ratio of active hydrogen atoms per epoxide group of from 0.01:1 to 0.95:1, more suitably from 0.05:1 to 0.8:1, most suitably from 0.1:1 to 0.5:1.

Another aspect of the present invention pertains to phenoxy type resin compositions prepared by the advancement reaction of (A) one or more of the polyglycidyl esters containing one or more mesogenic moieties, said polyglycidyl esters being those represented by either Formula I, with the proviso that the polyglycidyl ester of Formula I may include the polyglycidyl ester of 4,4'-dicarboxystilbene or the polyglycidyl ester or polymethylglycidyl ester of bis(4'-carboxyphenyl)-1,4-benzenediimine; or Formula II with

(B) at least one compound having an average of more than one active hydrogen atom per molecule; and wherein components (A) and (B) are employed in quantities which provide a ratio of active hydrogen atoms per epoxide group of from 0.96:1 to 1.05:1.

Another aspect of the present invention pertains to blends of (A) one or more of the polyglycidyl esters or monoglycidyl ester compounds containing one or more mesogenic moieties which polyglycidyl esters or monoglycidyl ester compounds are represented by the aforementioned Formulas I, II, III or IV with the proviso that the polyglycidyl ester of Formula I may include the polyglycidyl ester of 4,4'-dicarboxystilbene or the polyglycidyl ester or polymethylglycidyl ester of bis(4'-carboxyphenyl)-1,4-benzenediimine and the monoglycidyl ester of Formula III may include the monoglycidyl ester of 4-carboxybiphenyl, and (B) one or more polyepoxides represented by the following Formulas V, VI, VII, VIII, IX, X or XI;

Formula V

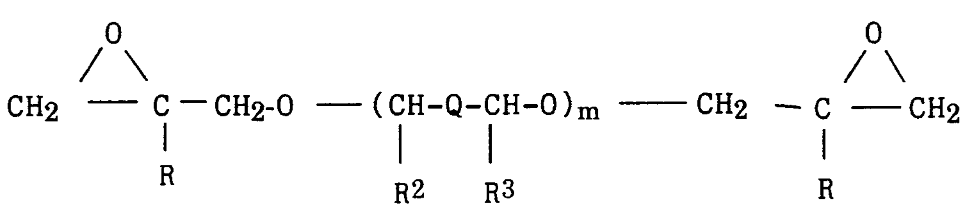

Formula VI

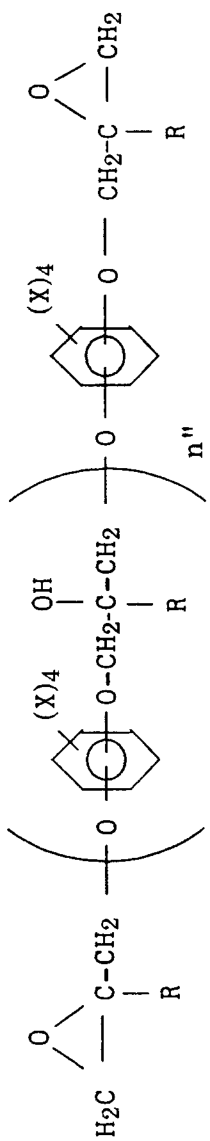

Formula VII

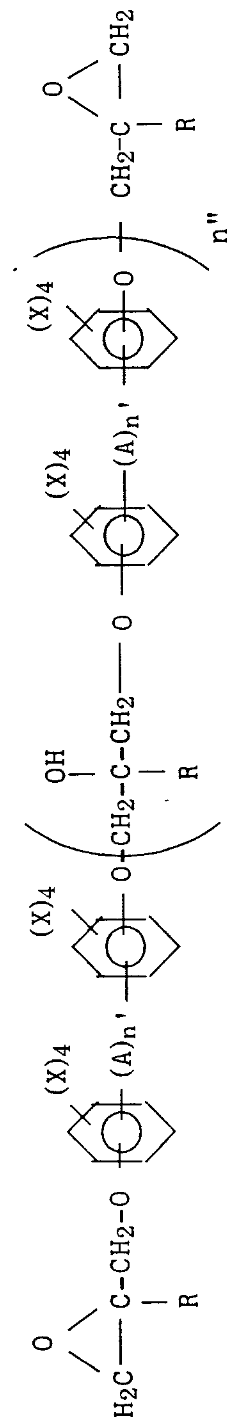

Formula VIII

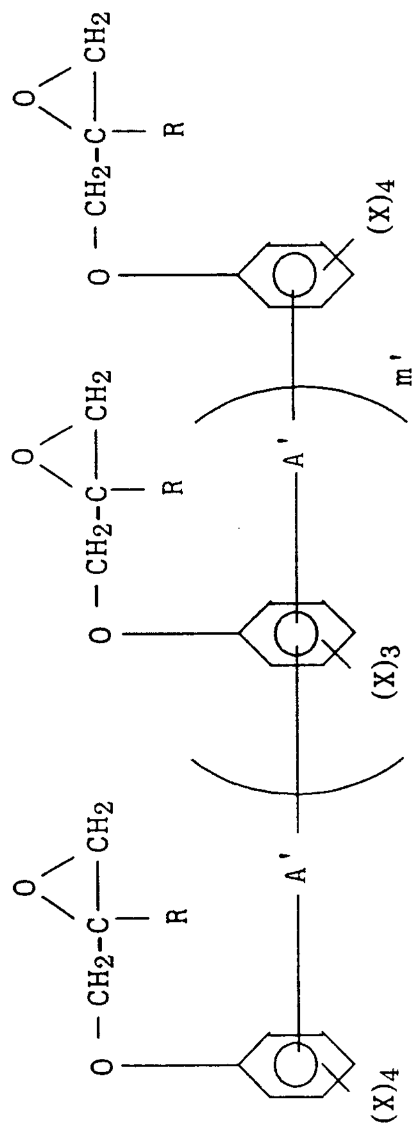

Formula IX

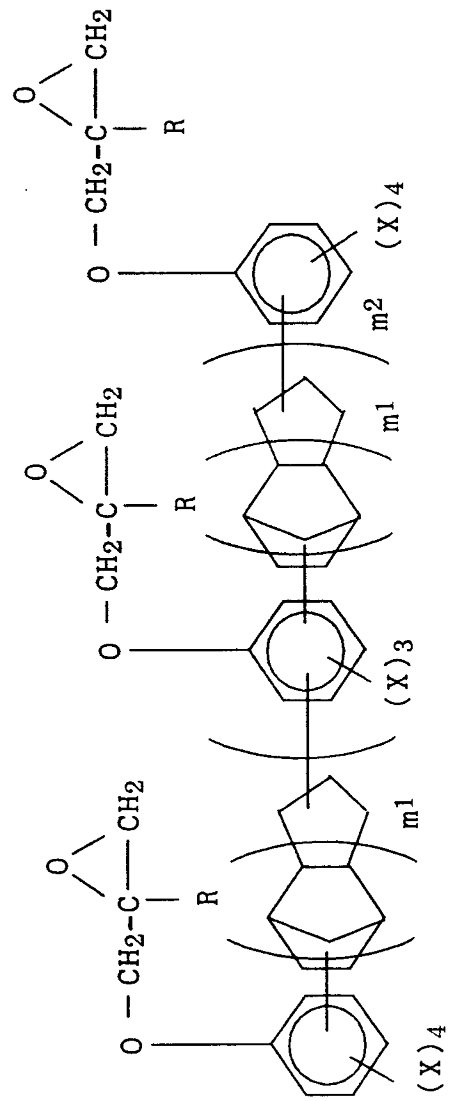

Formula X

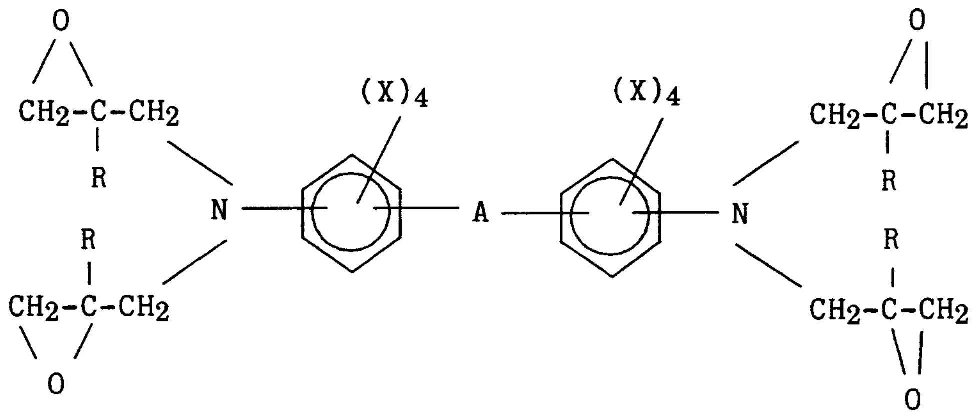

Formula XI

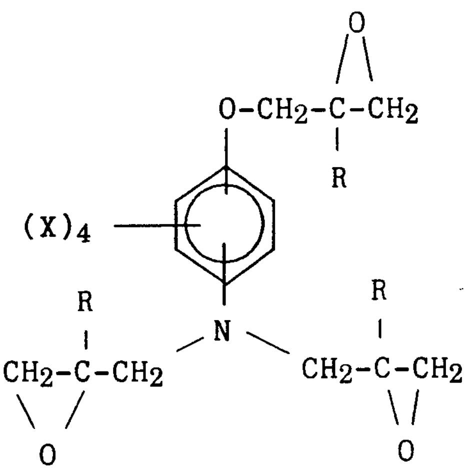

wherein each A is independently a divalent hydrocarbyl group having from 1 to 12, preferably from 1 to 6, more preferably from 1 to 3, carbon atoms, -O-, -S-, -S-S-, - SO-, $-SO_2-$, or -CO-; each A' is independently a divalent hydrocarbon group having from 1 to 6, preferably from 1 to 3, carbon atoms; Q is a single bond, $-CH_2-S-CH_2-$,

$$-(CH_2)_{n1}-,$$

or

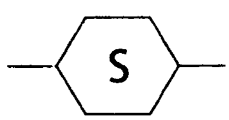 ;

each R is independently hydrogen or an alkyl group having from 1 to 4 carbon atoms; each $R^2$ and $R^3$ is independently hydrogen, a hydrocarbyl or halohydrocarbyl group having from 1 to 6, preferably from 1 to 3, more preferably from 1 to 2, carbon atoms; each X is independently hydrogen, a hydrocarbyl or hydrocarbyloxy group having from 1 to 12, preferably from 1 to 6, most preferably from 1 to 4, carbon atoms, a halogen atom, - $NO_2$ or C≡N; m has a value from 1 to 10, preferably from 1 to 4, more preferably from 1 to 2; m' has an average value from 0.01 to 12, preferably from 1 to 6, more preferably from 1 to 3; $m^1$ has an average value from 1 to 12, preferably from 1 to 6, more preferably from 1 to 3; $m^2$ has a value

from 1 to 12, preferably from 2 to 6, more preferably from 2 to 3; n' has a value of zero or 1; n'' has an average value from zero to 3, preferably from zero to 1.5, more preferably from zero to 0.5, and $n^1$ has an average value from 1 to 10; and wherein component (A) is present in an amount suitably from 1 to 99, more suitably from 10 to 80, most suitably from 10 to 50, percent by weight based upon the combined weight of components (A) and (B) and component (B) is present in an amount suitably from 99 to 1, more suitably from 90 to 20, most suitably from 90 to 50, percent by weight based upon the combined weight of components (A) and (B).

Another aspect of the present invention pertains to blends of (A) one or more of the advanced polyglycidyl esters containing one or more mesogenic moieties which advanced polyglycidyl esters are prepared by reacting one or more polyglycidyl esters represented by Formulas I or II, with the proviso that the polyglycidyl ester of Formula I may include the polyglycidyl ester of 4,4'-dicarboxystilbene or the polyglycidyl ester or polymethylglycidyl ester of bis(4'-carboxyphenyl)-1,4-benzenediimine, and at least one compound having an average of more than one active hydrogen atom per molecule; and (B) one or more polyepoxides represented by Formulas V, VI, VII, VIII, IX, X or XI; and wherein component (A) is present in an amount suitably from 1 to 99, more suitably from 10 to 80, most suitably from 10 to 50, percent by weight based upon the combined weight of components (A) and (B) and component (B) is present in an amount suitably from 99 to 1, more suitably from 90 to 20, most suitably from 90 to 50, percent by weight based upon the combined weight of components (A) and (B).

Another aspect of the present invention pertains to curable compositions comprising at least one polyglycidyl ester containing one or more mesogenic moieties represented by Formula I and a curing amount of a suitable curing agent therefor.

Another aspect of the present invention pertains to curable compositions comprising at least one polyglycidyl ester containing one or more mesogenic moieties represented by Formula II and a curing amount of a suitable curing agent therefor.

Another aspect of the present invention pertains to curable compositions comprising

(A) at least one polyglycidyl ester containing one or more mesogenic moieties, said polyglycidyl ester being represented by either Formula I, with the proviso that the polyglycidyl ester of Formula I may include the polyglycidyl ester of 4,4'-dicarboxystilbene or the polyglycidyl ester or polymethylglycidyl ester of bis(4'-carboxyphenyl)-1,4-benzenediimine; or Formula II;

(B) at least one of the aforementioned monoglycidyl ester compounds containing one or more mesogenic moieties, said monoglycidyl ester compounds being represented by Formulas III or IV, with the proviso that the monoglycidyl ester of Formula III may include the monoglycidyl ester of 4-carboxybiphenyl, and

(C) a curing amount of a suitable curing agent therefor; wherein component (A) is present in an amount suitably from 1 to 99, more suitably from 50 to 90, most suitably from 70 to 90, percent by weight based upon the combined weight of components (A) and (B) and component (B) is present in an amount suitably from 99 to 1, more suitably from 50 to 10, most suitably from 30 to 10, percent by weight based upon the combined weight of components (A) and (B).

Another aspect of the present invention pertains to curable compositions comprising

(A) an advanced polyglycidyl ester resulting from reacting

(1) at least one of the polyglycidyl esters containing one or more mesogenic moieties, said polyglycidyl esters being those represented by either Formula I, with the proviso that the polyglycidyl ester of Formula I may include the polyglycidyl ester of 4,4'-dicarboxystilbene or the polyglycidyl ester or polymethylglycidyl ester of bis(4'-carboxyphenyl)-1,4-benzenediimine; or Formula II; with

(2) at least one compound having an average of more than one active hydrogen atom per molecule; wherein components (A1) and (A2) are employed in quantities which provide a ratio of active hydrogen atoms to epoxide groups suitably from 0.01:1 to 0.95:1, more suitably from 0.05:1 to 0.8:1, most suitably from 0.1:1 to 0.5:1; and

(B) a curing amount of a suitable curing agent for component (A).

Another aspect of the present invention pertains to curable compositions comprising a blend of

(A) at least one of the polyglycidyl esters or monoglycidyl ester compounds containing one or more mesogenic moieties represented by Formulas I or II, with the proviso that the polyglycidyl ester of Formula I may include the polyglycidyl ester of 4,4'-dicarboxystilbene or the polyglycidyl ester or polymethylglycidyl ester of bis(4'-carboxyphenyl)-1,4-benzenediimine; or by the aforementioned Formulas III or IV with the proviso that the monoglycidyl ester of Formula III may include the monoglycidyl ester of 4-carboxybiphenyl;

(B) at least one of the polyepoxide resins represented by Formulas V, VI, VII, VIII, IX, X or XI; and

(C) a curing amount of a suitable curing agent therefor; wherein component (A) is present in an amount

suitably from 1 to 99, more suitably from 10 to 80, most suitably from 10 to 50, percent by weight based upon the combined weight of components (A) and (B) and component (B) is present in an amount suitably from 99 to 1, more suitably from 90 to 20, most suitably from 90 to 50, percent by weight based upon the combined weight of components (A) and (B).

Another aspect of the present invention pertains to curable compositions comprising a blend of

(A) at least one of the advanced polyglycidyl esters containing one or more mesogenic moieties prepared by reacting (1) one or more polyglycidyl esters represented by Formulas I or II, with the proviso that the polyglycidyl ester of Formula I may include the polyglycidyl ester of 4,4'-dicarboxystilbene or the polyglycidyl ester or polymethylglycidyl ester of bis(4'-carboxyphenyl)-1,4-benzenediimine; with (2) at least one compound having an average of more than one active hydrogen atom per molecule; wherein components (1) and (2) are employed in quantities which provide a ratio of active hydrogen atoms per epoxide group suitably from 0.01:1 to 0.95:1, more suitably from 0.05:1 to 0.8:1, most suitably from 0.1:1 to 0.5:1;

(B) at least one of the polyepoxide resins represented by Formulas V, VI, VII, VIII, IX, X or XI; and

(C) a curing amount of a suitable curing agent therefor; wherein component (A) is present in an amount suitably from 1 to 99, more suitably from 10 to 80, most suitably from 10 to 50, percent by weight based upon the combined weight of components (A) and (B) and component (B) is present in an amount suitably from 99 to 1, more suitably from 90 to 20, most suitably from 90 to 50, percent by weight based upon the combined weight of components (A) and (B).

A further aspect of the present invention pertains to products resulting from curing the aforementioned curable compositions.

A further aspect of the present invention pertains to products resulting from the application of an electric field or magnetic field or drawing and/or shear forces before and/or during curing or processing of the aforementioned compositions.

A still further aspect of the present invention pertains to products resulting from the application of an electric field or magnetic field or drawing and/or shear forces before and/or during curing or processing of a curable composition comprising (A) at least one polyglycidyl ester containing one or more mesogenic moieties said polyglycidyl esters being those represented by Formulas I or II, with the proviso that the polyglycidyl ester of Formula I may include the polyglycidyl ester of 4,4'-dicarboxystilbene or the polyglycidyl ester or polymethylglycidyl ester of bis(4'-carboxyphenyl)-1,4-benzenediimine; and (B) a curing amount of at least one suitable curing agent for component (A).

The term "mesogenic" as is used herein designates compounds containing one or more rigid rodlike structural units which have been found to favor the formation of liquid crystal phases in the case of low molar mass substances. Thus the mesogen or mesogenic moiety is that structure responsible for molecular ordering.

The mono and polyglycidyl ester compositions of the present invention can be prepared by any suitable method known to those skilled in the art. Suitable such methods include, for example, the following:

A. Reaction of a mono or polycarboxylic acid (or ester thereof) and an epihalohydrin to form the corresponding halohydrin ester of said mono or polycarboxylic acid followed by dehydrohalogenation of the resultant mono or polyhalohydrin ester.

B. Reaction of a salt, typically an alkali metal salt, of a mono or polycarboxylic acid and an epihalohydrin, typically epichlorohydrin.

C. Reaction of a mono or polycarboxylic acid halide and glycidol in the presence of a base, especially an organic tertiary amine.

D. Epoxidation of allyl esters of mono or polycarboxylic acids with peroxy compounds, especially organic peracids.

E. Transesterification of mono or polycarboxylic acid esters with glycidol in the presence of a catalyst.

F. Transesterification of mono or polycarboxylic acid esters with carboxylic acid glycidyl esters in the presence of a catalyst.

In the preparation of the mono or polyglycidyl ester of a mono or polycarboxylic acid, the carboxylic acid containing compound is typically reacted with an epihalohydrin in the presence of a suitable catalyst and in the presence or absence of a suitable solvent at a temperature suitably from 0°C to 150°C, more suitably from 20°C to 100°C, most suitably from 40°C to 80°C; at pressures suitably from 30 mm Hg vacuum to 100 psia., more suitably from 65 mm Hg vacuum to 50 psia., most suitably from atmospheric pressure to 20 psia.; and for a time sufficient to complete the reaction, usually from 1 to 48, more usually from 1 to 12, most usually from 1 to 6 hours. This initial reaction unless the catalyst is an alkali metal or alkaline earth metal hydroxide employed in stoichiometric quantities produces a halohydrin intermediate which is then reacted with a basic acting compound to convert the vicinal chlorohydrin groups to epoxide

groups. Reaction of the halohydrin intermediate and basic acting compounds in the presence or absence of a suitable solvent is typically conducted at a temperature suitably from 0°C to 100°C, more suitably from 20°C to 80°C, most suitably from 25°C to 60°C; at pressures suitably from 30 mm Hg vacuum to 100 psia., more suitably from 45 mm Hg vacuum to 50 psia., most suitably from 60 mm Hg vacuum to atmospheric pressure; and for a time sufficient to complete the dehydrohalogenation reaction, usually from 15 minutes to 12 hours, more usually from 30 minutes to 6 hours, most usually from 1 hour to 4 hours. The resultant product is a glycidyl ester compound.

Suitable epihalohydrins which can be employed to prepare the mono and polyglycidyl esters of the present invention include, for example, those represented by the following Formula XII

Formula XII

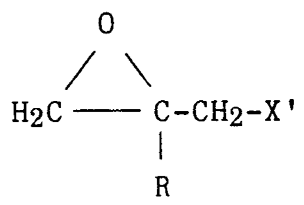

wherein R is as previously defined; and X' is a halogen. Particularly suitable such epihalohydrins include, for example, epichlorohydrin, epibromohydrin, epiiodohydrin, methylepichlorohydrin, methylepibromohydrin, methylepiiodohydrin, or any combination thereof.

Suitable carboxylic acid containing compounds which can be employed to prepare the mono and polyglycidyl esters of the present invention include, for example, those represented by the following Formulas XIII, XIV, XV or XVI

Formula XIII

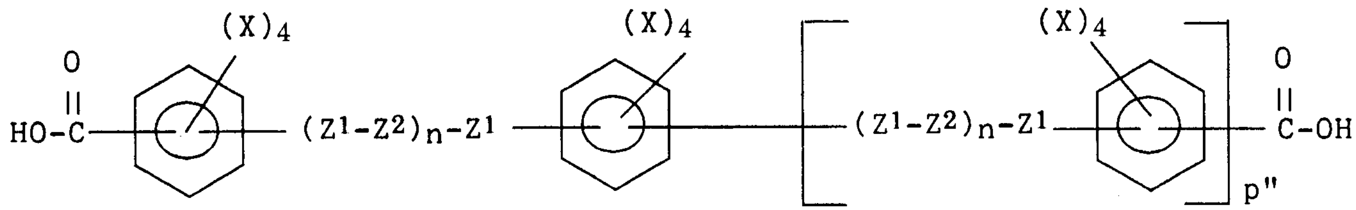

Formula XIV

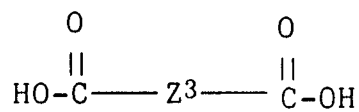

Formula XV

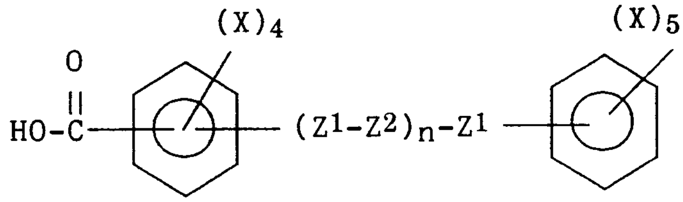

Formula XVI

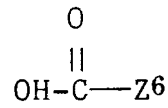

wherein at least 80 percent of the $-(Z^1-Z^2)_n-Z^1$-linkages and the carboxylic acid groups are in the para position with respect to each other; wherein $R^1$, X, $X^1$, $Z^1$, $Z^2$, $Z^3$, $Z^4$, $Z^6$, n, n', and p'' are as previously defined.

Particularly suitable carboxylic acid containing compounds include, for example, 4,4'-dicarboxy-α-methylstilbene, 4,4'-dicarboxybenzanilide, 4,4'-dicarboxy-2,2'-dimethylazoxybenzene, 4,4'-dicarboxystilbene, 4,4'-dicarboxyazobenzene, 4,4'-dicarboxyazoxybenzene, 4,4'-dicarboxy-α-cyanostilbene, 4,4'-dicarboxydiphenylacetylene, N,N'-bis(4-carboxyphenyl)terephthalamide, 4,4'-dicarboxy-3,3',5,5'-tetramethylstilbene, 4,4'-dicarboxy-3,3',5,5'-tetrabromostilbene, 4,4'-dicarboxy-3,3',5,5'-tetramethyl-α-methylstilbene, N-biphenyl-4-carboxybenzamide, N-2-naphthyl-4-carboxybenzamide, N-phenyl-4-carboxybenzamide, N-(4'-carboxyphenyl)benzamide, 4-carboxystilbene, 4-carboxy-α-methylstilbene, 4-carboxyazobenzene, 4-carboxy-α-cyanostilbene, 4-carboxyazoxybenzene, 4,4'-dicarboxydiphenylazomethine, or any combination thereof.

Suitable catalysts which can be employed to prepare the mono and polyglycidyl esters of the present invention include, for example, ammonium halides such as, for example, benzyltrimethylammonium chloride, benzyltrimethylammonium bromide, tetrabutylammonium chloride, tetrabutylammonium bromide, tetraoctylammonium chloride, tetraoctylammonium bromide, tetramethylammonium chloride, tetramethylammonium bromide, or any combination thereof.

Suitable basic acting compounds which can be employed to prepare the mono and polyglycidyl esters of the present invention include, for example, alkali metal or alkaline earth metal hydroxides, carbonates, bicarbonates or any combination thereof. Particularly suitable such compounds include, for example, sodium hydroxide, potassium hydroxide, lithium hydroxide, calcium hydroxide, barium hydroxide, magnesium hydroxide, manganese hydroxide, sodium carbonate, potassium carbonate, lithium carbonate, calcium carbonate, barium carbonate, magnesium carbonate, manganese carbonate, sodium bicarbonate, potassium bicarbonate, lithium bicarbonate, calcium bicarbonate, barium bicarbonate, magnesium bicarbonate, manganese bicarbonate, or any combination thereof. Most preferred is sodium hydroxide or potassium hydroxide.

Suitable solvents which can be employed herein include, for example, alcohols, aliphatic hydrocarbons, aromatic hydrocarbons, glycol ethers, amides, sulfoxides, sulfones, or any combination thereof. Particularly suitable solvents include, for example, methanol, ethanol, isopropanol, hexane, heptane, octane, nonane, decane, toluene, xylene, ethylene glycol methyl ether, ethylene glycol ethyl ether, ethylene glycol n-butyl ether, ethylene glycol phenyl ether, propylene glycol methyl ether, propylene glycol phenyl ether, tripropylene glycol methyl ether, diethylene glycol methyl ether, diethylene glycol ethyl ether, diethylene glycol n-butyl ether, diethylene glycol phenyl ether, butylene glycol methyl ether, N,N-dimethylformamide, N-methylpyrrolidinone, N,N-dimethylacetamide, dimethylsulfoxide, sulfolane, or any combination thereof.

The solvent, if used, is usually employed in amounts suitably from 5 to 95, more suitably from 20 to 60, most suitably from 30 to 40, percent by weight based upon the combined weight of solvent and epihalohydrin.

Suitable compounds having an average of more than one active hydrogen atom per molecule which can be employed to prepare the advanced resin compositions of the present invention include, for example, bisphenols, thiobisphenols, dicarboxylic acids and compounds containing one primary amine or amide group or two secondary amine groups such as those represented by the following Formulas XVII or XVIII;

Formula XVII

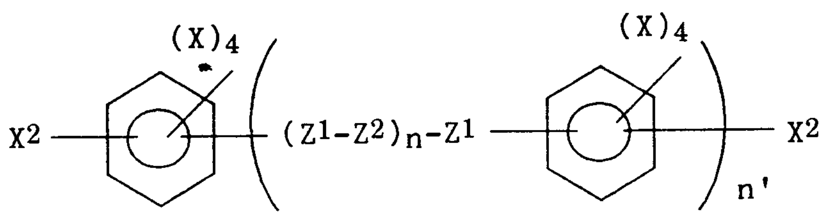

Formula XVIII

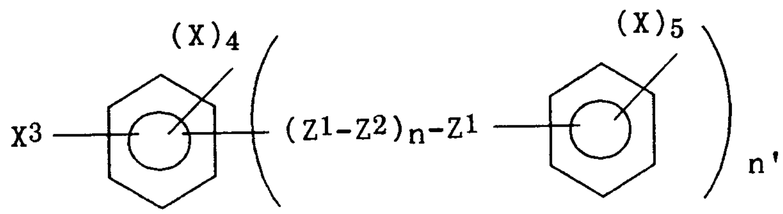

wherein $X^2$ is independently a hydroxyl, carboxylic acid, -SH, or $-NHR^2$ group; $R^2$ is an alkyl group having from 1 to 4 carbon atoms; $X^3$ is $NH_2$, $NH_2-SO_2-$, $NH_2-CO-$, or $NH_2-Z^5-O-$; $Z^5$ is an alkyl or cycloalkyl group having from 1 to 12 carbon atoms; and wherein $Z^1$, X, Z', $R^1$, $Z^2$, n and n' are as hereinbefore defined.

The advancement of the polyglycidyl esters containing one or more mesogenic moieties with compounds having an average of more than one active hydrogen per molecule is employed to linearly chain extend the resin. This linear chain extension is required for some mesogen-containing resin compositions in order to obtain liquid crystal character. The advancement of the mesogenic polyglycidyl ester resins can also be used to increase the temperature range in which a particular resin is liquid crystalline and to control the degree of crosslinking during the final curing stage.

The polyglycidyl ester containing one or more mesogenic moieties and the compound having an average of more than one active hydrogen atom per molecule are reacted in amounts which provide suitably from 0.01:1 to 0.95:1, more suitably from 0.05:1 to 0.9:1, most suitably from 0.10:1 to 0.50:1 active hydrogen atoms per epoxy group.

Particularly suitable compounds having an average of more than one active hydrogen atom per molecule which can be employed herein include hydroxyl-containing compounds, carboxylic acid-containing compounds and primary amine-containing compounds. These compounds include, for example, those represented by Formulas XVII and XVIII.

Particularly suitable hydroxyl-containing compounds include, for example, hydroquinone, bisphenol A, 4,4'-dihydroxydiphenylmethane, 4,4'-thiodiphenol, 4,4'-sulfonyldiphenol, 4,4'-dihydroxydiphenyl oxide, 4,4'-dihydroxybenzophenone, 1,1-bis(4-hydroxyphenyl)-1-phenylethane, 3,3',5,5'-tetrachorobisphenol A, 3,3'-dimethoxybisphenol A, 4,4'-dihydroxybiphenyl, 4,4'-dihydroxy-$\alpha$,$\alpha$'-diethylstilbene, 4,4'-dihydroxy-$\alpha$-methylstilbene, 4,4'-dihydroxybenzanilide, 4,4'-dihydroxy-2,2'-dimethylazoxybenzene, 4,4'-dihydroxy-$\alpha$-cyanostilbene, bis(4-hydroxyphenyl)terephthalate, N,N'-bis(4-hydroxyphenyl)terephthalamide, bis(4'-hydroxybiphenyl)terephthalate, 4,4'-dihydroxyphenylbenzoate, bis(4'-hydroxyphenyl)-1,4-benzenediimine, 4,4"-dihydroxybiphenylbenzoate, 1,4-bis(4'-hydroxyphenyl-1'-carboxamide)benzene, 1,4-bis(4'-hydroxyphenyl-1'-carboxy)benzene, 4,4'-bis(4"-hydroxyphenyl-1"-carboxy)biphenyl, or any combination thereof.

Particularly suitable carboxylic acid-containing compounds include, for example, terephthalic acid, 4,4'-benzanilide dicarboxylic acid, 4,4'-phenylbenzoate dicarboxylic acid, 4,4'-stilbenedicarboxylic acid, 4,4'-dicarboxybiphenyl, 4,4'-dicarboxydiphenylazomethine, or any combination thereof.

Particularly suitable primary amine-containing compounds include, for example, aniline, 4'-sulfonamido-N-phenyl benzamide, 4'-sulfonamido-N'-phenyl-4-chlorobenzamide, 4-amino-1-phenylbenzoate, 4-amino-N-phenylbenzamide, N-phenyl-4-aminophenyl-1-carboxamide, phenyl-4-aminobenzoate, biphenyl-4-aminobenzoate, 1-phenyl-4'-aminophenylterephthalate, or any combination thereof.

The advancement reaction can be conducted in the presence of a suitable advancement catalyst such as, for example, phosphines, quaternary ammonium compounds, phosphonium compounds, tertiary amines

or any combination thereof. Particularly suitable catalysts include, for example, ethyltriphenylphosphonium chloride, ethyltriphenylphosphonium bromide, ethyltriphenyl-phosphonium iodide, ethyltriphenylphosphonium diacetate (ethyltriphenylphosphonium acetate•acetic acid complex), ethyltriphenylphosphonium phosphate, tetrabutylphosphonium chloride, tetrabutylphosphonium bromide, tetrabutylphosphonium iodide, tetrabutylphosphonium diacetate (tetrabutylphosphonium acetate•acetic acid complex), butyltriphenylphosphonium tetrabromobisphenate, butyltriphenylphosphonium bisphenate, butyltriphenylphosphonium bicarbonate, benzyltrimethylammonium chloride, tetramethylammonium hydroxide, triethylamine, tripropylamine, tributylamine, 2-methylimidazole, benzyldimethylamine, or any combination thereof.

The amount of advancement catalyst depends, of course, upon the particular reactants and catalyst employed; however, it is usually employed in quantities of from 0.03 to 3, preferably from 0.03 to 1.5, most preferably from 0.05 to 1.5 percent by weight based upon the weight of the epoxy-containing compound.

The advancement reaction can be conducted at atmospheric, superatmospheric or subatmospheric pressures at temperatures of from 20°C to 260°C, preferably from 80°C to 240°C, more preferably from 100°C to 200°C. The time required to complete the advancement reaction depends upon the temperature employed. Higher temperatures require shorter periods of time whereas lower temperatures require longer periods of time. Generally, however, times of from 5 minutes to 24 hours, preferably from 30 minutes to 8 hours, more preferably from 30 minutes to 3 hours are suitable.

If desired, the advancement reaction can be conducted in the presence of one or more solvents. Suitable such solvents include, for example, glycol ethers, aliphatic and aromatic hydrocarbons, aliphatic ethers, cyclic ethers, ketones, esters, amides, or any combination thereof. Particularly suitable solvents include, for example, toluene, benzene, xylene, methyl ethyl ketone, methyl isobutyl ketone, diethylene glycol methyl ether, dipropylene glycol methyl ether, dimethylformamide, dimethyl-sulfoxide, N-methylpyrrolidinone, tetrahydrofuran, propylene glycol methyl ether, or any combination thereof. The solvents can be employed in amounts of from zero to 80%, preferably from 20% to 60%, more preferably from 30% to 50% by weight based upon the weight of the reaction mixture.

When the polyglycidyl ester containing one or more mesogenic moieties and the compound having an average of more than one active hydrogen atom per molecule are reacted in amounts which provide from 0.96:1 to 1.05:1 active hydrogen atoms per epoxy group, a relatively high molecular weight substantially thermoplastic resinous product is produced. These thermoplastic resin compositions contain little, if any, curable residual epoxide functionality and may even contain an active hydrogen functionality, depending upon which component is employed in excess, the polyglycidyl ester or the active hydrogen containing compound. These phenoxy type resins may thus be processed using the typical processing methods employed with conventional thermoplastic resins, such as, for example, injection molding or extrusion. Thermosetting may, however, be induced, for example, via reaction of all or a part of the backbone secondary aliphatic hydroxyl groups produced in the aforesaid advancement reaction, with a curing agent therefor. One class of suitable curing agents includes, for example, the di or polyisocyanates, as well as the blocked di or polyisocyanates which can be induced to react with the secondary hydroxyl groups providing urethane crosslinks between the resin chains. An example of a specific diisocyanate especially useful herein is 4,4'-diisocyanatodiphenylmethane. When the compound having an average of more than one active hydrogen atom per molecule used in the advancement reaction is a diphenol, the resultant resinous product is a phenoxy resin. If desired, the reaction can be conducted in the presence of a suitable catalyst such as, for example, those catalysts described herein for use in the advancement reaction.

The compositions of the present invention containing an average of more than one vicinal epoxy group per molecule can be cured with any suitable curing agent for curing epoxy-containing resins such as, for example, primary and secondary polyamines, carboxylic acids and anhydrides thereof, aromatic hydroxyl containing compounds, imidazoles, guanidines, urea-aldehyde resins, melamine-aldehydes resins, alkoxylated urea-aldehyde resins, alkoxylated melamine-aldehyde resins, aliphatic amines, cycloaliphatic amines, aromatic amines, or any combination thereof. Particularly suitable curing agents include, for example, methylene dianiline, dicyandiamide, ethylene diamine, diethylenetriamine, triethylenetetramine, tetraethylenepentamine, urea-formaldehyde resins, melamine-formaldehyde resins, methylolated urea-formaldehyde resins, methylolated melamine-formaldehyde resins, phenol-formaldehyde novolac resins, cresol-formaldehyde novolac resins, sulfanilamide, diaminodiphenylsulfone, diethyltoluenediamine, t-butyltoluenediamine, bis-4-aminocyclohexylmethane, isophoronediamine, diaminocyclohexane, hexamethylenediamine, piperazine, aminoethylpiperazine, 2,5-dimethyl-2,5-hexanediamine, 1,12-dodecanediamine, tris-3-aminopropylamine, or any combination thereof.

The curing agents are employed in amounts which will effectively cure the composition; however, these amounts will depend upon the particular polyglycidyl ester and curing agent employed. Generally, suitable

amounts include, for example, from 0.95:1 to 1.2:1 equivalents of curing agent per equivalent of polyglycidyl ester.

The monoglycidyl esters containing one or more mesogenic moieties of the present invention can be employed as reactive diluents for the polyglycidyl esters of the present invention as well as for polyglycidyl esters substantially free of mesogenic moieties, or epoxy resins. For polyglycidyl esters free of mesogenic moieties, the monoglycidyl esters provide a means of incorporating mesogenic moieties into the composition so as to enhance one or more properties when cured.

The mesogenic polyglycidyl esters of the present invention can also be employed for the purpose of improving the properties of epoxy resins substantially free of mesogenic moieties. Generally, suitable amounts of mesogenic polyglycidyl esters are from 1 to 99, more suitably from 10 to 80, most suitably from 10 to 50 weight percent based on the total weight of the combined resins. Representative of the epoxy resins free of mesogenic moieties include, for example, the diglycidyl ethers of resorcinol, bisphenol A, 4,4'-dihydroxydiphenylmethane, 3,3',5,5'-tetrabromobisphenol A, 4,4'-thiodiphenol, 4,4'-sulfonyldiphenol, 4,4'-dihydroxydiphenyl oxide, 4,4'-dihydroxybenzophenone, 1,1-bis(4-hydroxyphenyl)-1-phenylethane, 3,3',5,5'-tetrachlorobisphenol A, 3,3'-dimethoxybisphenol A; the triglycidyl ether of tris(hydroxyphenyl)methane; the polyglycidyl ether of a phenol or substituted phenol-aldehyde condensation product (novolac); the polyglycidyl ether of a dicyclopentadiene or an oligomer thereof and phenol condensation product; the advancement reaction products of the aforesaid di- and polyglycidyl ethers with aromatic di- or polyhydroxyl- or carboxylic acid- containing compounds including, for example, bisphenol A (4,4'-isopropylidenediphenol), o-, m-, p-dihydroxybenzene, 2,4-dimethylresorcinol, 4-chlororesorcinol, tetramethylhydroquinone, 1,1-bis(4-hydroxyphenyl)ethane, 1,1-bis(4-hydroxyphenyl)-1-phenylethane, 4,4'-dihydroxydiphenyl ether, 3,3',5,5'-tetramethyldihydroxydiphenyl ether, 3,3',5,5'-dichlorodihydroxydiphenylether, 4,4'-bis(p-hydroxyphenyl isopropyl)diphenyl ether, 4,4'-bis(p-hydroxyphenoxy)benzene, 4,4'-bis(p-hydroxyphenoxy)diphenyl ether, 4,4'-bis(4(4-hydroxyphenoxy)phenyl sulfone)diphenyl ether, 4,4'-dihydroxydiphenyl sulfone, 4,4'-dihydroxydiphenyl sulfide, 4,4'-dihydroxydiphenyl disulfide, 2,2'-dihydroxydiphenyl sulfone, 4,4'-dihydroxydiphenyl methane, 1,1-bis(p-hydroxyphenyl)cyclohexane, 4,4'-dihydroxybenzophenone, phloroglucinol, pyrogallol, 2,2',5,5'-tetrahydroxydiphenyl sulfone, tris(hydroxyphenyl)methane, dicyclopentadiene diphenol, tricyclopentadiene diphenol; or any combination thereof.

Before and/or during processing and/or curing of the polyglycidyl ester compositions into a part, electric or magnetic fields or shear stresses can be applied for the purpose of orienting the liquid crystal moieties contained or developed therein which in effect improves the mechanical properties. As specific examples of these methods, Finkelmann, et al, Macromol. Chem., 180, 803-806 (March 1979) induced orientation in thermotropic methacrylate copolymers containing mesogenic side chain groups decoupled from the main chain via flexible spacers in an electric field. Orientation of mesogenic side chain groups decoupled from the polymer main chain via flexible spacers in a magnetic field has been demonstrated by Roth and Kruecke, Macromol. Chem., 187, 2655-2662 (November 1986). Magnetic field induced orientation of mesogenic main chain containing polymers has been demonstrated by Moore, et al, ACS Polymeric Material Sciences and Engineering, 52, 84-86 (April-May 1985). Magnetic and electric field orientation of low molecular weight mesogenic compounds is discussed by W. R. Krigbaum in Polymer Liquid Crystals, pages 275-309 (1982) published by Academic Press, Inc. All of the above are incorporated herein by reference in their entirety.

In addition to orientation by electric or magnetic fields, polymeric mesophases can be oriented by shear forces which are induced by drawing and/or flow through dies, orefices, and mold gates. A general discussion for orientation of thermotropic liquid crystal polymers by this method is given by S. K. Garg and S. Kenig in High Modulus Polymers, pages 71-103 (1988) published by Marcel Dekker, Inc. For the mesomorphic systems based on the polyglycidyl ester compositions, this shear orientation can be produced by processing methods such as injection molding, extrusion, pultrusion, filament winding, filming and prepreging.

The mesogenic polyglycidyl ester of the present invention can be blended with other materials such as solvents or diluents, fillers, pigments, dyes, flow modifiers, thickeners, reinforcing agents, mold release agents, wetting agents, stabilizers, fire retardant agents, surfactants, or any combination thereof.

These additives are added in functionally equivalent amounts, e.g., the pigments and/or dyes are added in quantities which will provide the composition with the desired color; however, they are suitably employed in amounts of from zero to 20, more suitably from 0.5 to 5, most suitably from 0.5 to 3 percent by weight based upon the weight of the total blended composition.

Solvents or diluents which can be employed herein include, for example, hydrocarbons, ketones, glycol ethers, aliphatic ethers, cyclic ethers, esters, amides, or any combination thereof. Particularly suitable solvents or diluents include, for example, toluene, benzene, xylene, methyl ethyl ketone, methyl isobutyl

ketone, diethylene glycol methyl ether, dipropylene glycol methyl ether, dimethylformamide, N-methylpyrrolidinone, tetrahydrofuran, propylene glycol methyl ether, or any combination thereof.

The modifiers such as thickeners and flow modifiers can be suitably employed in amounts of from zero to 10, more suitable from 0.5 to 6, most suitably from 0.5 to 4 percent by weight based upon the weight of the total composition.

Reinforcing materials which can be employed herein include natural and synthetic fibers in the form of woven fabric, mats, monofilament, multifilament, unidirectional fibers, rovings, random fibers or filaments, inorganic fillers or whiskers, or hollow spheres. Suitable reinforcing materials include, glass, ceramics, nylon, rayon, cotton, aramid, graphite, polyalkylene terephthalates, polyethylene, polypropylene, polyesters, or any combination thereof.

Suitable fillers which can be employed herein include, for example, inorganic oxides, ceramic microspheres, plastic microspheres, glass microspheres, inorganic whiskers, $CaCO_3$, or any combination thereof.

The fillers can be employed in amounts suitable from zero to 95, more suitably from 10 to 80, most suitable from 40 to 60 percent by weight based upon the weight of the total composition.

The following examples were illustrative of the present invention, but were not to be construed as to limiting its scope in any manner.

EXAMPLE 1

A. Synthesis of 4,4',$\alpha$,$\beta$-Tetrabromodiphenylethane

$\alpha$,$\beta$-Diphenylethane (60.0 grams, 0.329 mole) and acetic acid (660 milliliters) were added to a one liter glass resin kettle reactor and stirred at 22° C to provide a solution. A mixture of bromine (82 milliliters) and deionized water (32 milliliters) was added to the solution in the reactor and heating commenced. After twenty five minutes, a reflux was achieved at a 103° C temperature. At this time, crystalline white product was observed suspended in the reactor. After 45 minutes, the reflux temperature has reached 108° C and extensive white crystalline product was observed suspended in the reactor. The product was recovered by filtration through a coarse fritted glass funnel, washed with acetic acid (200 milliliters) then washed with diethylether (50 milliliters). After drying in a vacuum oven at 60° C, a constant weight of 56.98 grams of white crystalline powder was obtained.

B. Synthesis of 4,4'-Dicyanostilbene

4,4',$\alpha$,$\beta$-Tetrabromodiphenylethane from A above (56.90 grams, 0.114 mole), cuprous cyanide (63.73 grams, 0.712 mole) and pyridine (64.0 milliliters) were added to a one liter glass resin kettle reactor and stirred as a powder with heating. Once the temperature reached 148° C, a black solution formed. After an additional thirty minutes, the reaction temperature reached 205° C and was held therein for an additional eighty three minutes. After this time, additional pyridine (136.6 milliliters) was added to the reactor and causes the temperature to decrease to 132° C. This temperature was maintained for five minutes, then the product was poured into a beaker containing stirred concentrated hydrochloric acid (341.4 milliliters) over a twelve minute period. The resultant black slurry was filtered while at 87° C to provide a black powder product which was washed with additional concentrated hydrochloric acid (113.8 milliliters), then with deionized water (100 milliliters). The gray brown powder product was recovered then dried in a forced air oven at 100° C to a constant weight of 37.12 grams. Recrystallization was completed by boiling the crude product in nitrobenzene (175 milliliters) followed by filtration through a coarse fritted glass funnel to remove a black insoluble residue. The filtrate was stored at 4° C for twelve hours then the crystalline product was recovered by filtration. After drying at ambient temperature (23° C to 25° C) for 48 hours, 14.3 grams (slight odor of nitrobenzene still present) of shimmering light brown needles of 4,4'-dicyanostilbene were obtained.

C. Synthesis of Diiminoethylether dihydrochloride of 4,4'-Dicyanostilbene

4,4'-Dicyanostilbene from B above (14.3 grams), and nitrobenzene (572 milliliters) were combined in a beaker and brought to a boil (208° C) with stirring. The resultant solution was added to a one liter glass resin kettle reactor and stirred with cooling. After twelve minutes, the temperature reached 25° C, and anhydrous ethanol (35.8 milliliters) was added to the reactor. After an additional ten minutes, the reaction temperature reached 0° C and sparging with anhydrous hydrogen chloride commenced. Two minutes after beginning the sparge, the temperature exotherms to 9° C while maintaining the cooling bath on the reactor at - 52° C. After an additional nine minutes, the temperature decreases to -1° C and sparging was

terminated. After an additional seven minutes, the temperature decreases to - 2° C and the cooling bath was removed from the reactor and the contents therein allowed to warm to room temperature (24° C). After 48 hours, the reactor contents were filtered through a coarse fritted glass funnel. The light yellow powder retained on the funnel was washed with anhydrous diethylether (75 milliliters) to provide 31.56 grams (slightly wet) diiminoethylether dihydrochloride product.

D. Synthesis of Diethyl-4,4'-stilbenedicarboxylate

Diiminoethylether dihydrochloride of 4,4'-dicyanostilbene from C above (31.56 grams, slightly wet) and deionized water (95 milliliters) were added to a one liter glass resin kettle reactor and stirred with heating. After twenty five minutes, the temperature reached 100° C and was held therein for one hour. The product was recovered by filtration on paper as a light tan colored powder then dried at ambient temperature (24° C) for twelve hours to provide 19.4 grams (slightly wet) diethyl-4,4'-stilbenedicarboxylate.

E. Hydrolysis of Diethyl-4,4'-stilbenedicarboxylate

Diethyl-4,4'-stilbenedicarboxylate from D above (19.4 grams, slightly wet), ethylene glycol (323 grams), deionized water (81 grams) and sodium hydroxide (40.4 grams) were added to a resin kettle reactor and stirred with heating. After nineteen minutes, a reflux was achieved at 126° C and was held therein for six hours. The reaction product was diluted into deionized water (3 liters) and the resultant solution passed through a filter. The filtrate was added to a beaker, stirred and acidified to a pH of one with concentrated hydrochloric acid. The resultant gelatinous white slurry was heated to 80° C then filtered through paper to recover a white powder product. After washing with deionized water (100 milliliters), the product was dried in a forced air oven at 80° C for fourteen hours to a constant weight of 14.4 grams. Fourier transform infrared spectrophotometric analysis of a potassium chloride pellet confirmed the product structure for 4,4'-stilbenedicarboxylic acid.

F. Epoxidation of 4,4'-Stilbenedicarboxylic Acid

4,4'-Stilbenedicarboxylic acid (13.41 grams, 0.10 -COOH equivalent) from E above, epichlorohydrin (231.33 grams, 2.5 mole) and tetrabutylammonium chloride (0.134 gram, 1.0 % wt. of the diacid reactant used) were added to a one liter glass round bottom reactor and heated to 60° C with magnetically driven stirring under a nitrogen atmosphere flowing at a rate of one liter per minute. After twenty hours at the 60° C reaction temperature, fourier transform infrared spectrophotometric analysis demonstrated incomplete conversion of the carboxylic acid groups (acid carbonyl absorbance at 1682 $cm^{-1}$) to ester groups (ester carbonyl absorbance at 1716 $cm^{-1}$) hence the reaction temperature was increased to 80° C. After 156 minutes at the 80° C reaction temperature, fourier transform infrared spectrophotometric analysis demonstrated complete conversion of the carboxylic acid groups to ester groups concurrent with the formation of a hazy, light brown colored solution. At this time, a water separator was interspersed between the reactor and the chilled (-2.5° C) glycol condenser and an addition funnel containing sodium hydroxide (4.5 grams, 0.113 mole) dissolved in deionized water (5.5 grams, 55 % wt. of the solution) and a vacuum line were added to the reactor. The nitrogen purge was shut off simultaneous with initiation of the vacuum. The vacuum and reaction temperature were equilibrated at 84 mm Hg and 60° C, respectively and such that a vigorous reflux was maintained with continuous return of dry epichlorohydrin from the water separator to the reactor. After equilibration, dropwise addition of the aqueous sodium hydroxide commenced accompanied by a gradual reduction in vacuum and reaction temperature. After 42 minutes, addition of the aqueous sodium hydroxide was complete and vacuum and reaction temperature were at 65 mm Hg and 55° C, respectively. After an additional 2 hours at the 65 mm Hg vacuum and 55° C reaction temperature, heating ceases and the product slurry was cooled to 50° C. The recovered slurry was filtered under a nitrogen atmosphere and the resultant light amber colored solution rotary evaporated under a vacuum (5 mm Hg final conditions) at 90° C for 30 minutes. The product was recovered (14.0 grams) as a white powder. Titration of a portion of the product revealed an epoxide equivalent weight of 192.81. Fourier transform infrared spectrophotometric analysis of a potassium chloride pellet of the product confirmed the product structure for the diglycidyl ester of 4,4'-stilbenedicarboxylic acid (ester carbonyl absorbance at 1716 $cm^{-1}$, epoxide - C-O- stretching absorbance at 852 and 906 $cm^{-1}$).

G. Characterization of Liquid Crystallinity in the Diglycidyl Ester of 4,4'-Stilbenedicarboxylic Acid

Analysis of the diglycidyl ester of 4,4'-stilbenedicarboxylic acid from F above via crosspolarized light microscopy was completed using a optical microscope equipped with a programmable hot stage using a heating rate of 10 ° C per minute. The results are reported in Table I.

TABLE I

CROSSPOLARIZED LIGHT MICROSCOPY ANALYSIS OF THE DIGLYCIDYL ESTER OF 4,4'-STILBENEDICARBOXYLIC ACID

| Cycle Designation | Observed Transition Temperatures (°C) | Comments |
|---|---|---|
| First heating | 30 | Birefringent crystalline solid. |
| | 129 | First fluidity noted. |
| | 135 | Isotropization completed. |
| First cooling | 106 | First mobile nematic texture formed. |
| | 92 | First crystallization noted. |
| Second heating | 30 | Birefringent crystalline solid. |
| | 132 | Isotropization completed. |
| Second cooling | 106 | First mobile nematic texture formed. |
| | 88 | First crystallization noted. |

The diglycidyl ester was a monotropic liquid crystal with a nematic texture.

Analysis of a portion (12.5 milligrams) of the diglycidyl ester of 4,4'-stilbenedicarboxylic acid from F above via differential scanning calorimetry was completed using a heating and cooling rate of 5 ° C per minute under nitrogen flowing at 35 cubic centimeters per minute over a temperature range of 35 ° C to 160 ° C. The results are reported in Table II.

TABLE II

DIFFERENTIAL SCANNING CALORIMETRY ANALYSIS OF THE DIGLYCIDYL ESTER OF 4,4'-STILBENEDICARBOXYLIC ACID

| Cycle Designation | Observed Transition Temperatures (°C) peak/range | Enthalpy (J/G) | Comments |
|---|---|---|---|
| First heating | 131/105-140 | 77.8 | Endotherm |
| First cooling | 104/107-100<br>92/95-88<br>69/80-55 | 0.74<br>0.70<br>31.3 | Exotherm<br>Exotherm<br>Exotherm |
| Second heating | 128/110-135 | 51.7 | Endotherm |
| Second cooling | 105/108-100<br>90/92-87 | 0.81<br>0.44 | Exotherm<br>Exotherm |

H. Preparation of a Cured Casting of the Diglycidyl Ester of 4,4'-Stilbenedicarboxylic Acid and Evaluation of Susceptibility to Shear Induced Orientation During Cure

A portion (0.5155 gram, 0.00268 epoxide equivalent) of the diglycidyl ester of 4,4'-stilbenedicarboxylic acid from F above and sulfanilamide (0.1152 gram, 0.00268 amine equivalent) was combined and ground together to form a homogeneous powder mixture. Differential scanning calorimetry analysis of a portion (8.2 milligrams) of the powder heated at 10°C per minute under nitrogen flowing at 35 cubic centimeters per minute revealed an exotherm (195 joules per gram) between 125 and 225°C. A portion of the powder was placed between two glass plates and heated at 20°C per minute to 125°C at which point an isotropic melt was observed via optical microscopy (70X magnification) under crosspolarized light. Following formation of the isotropic melt, the resin was cooled to 120°C. A nematic liquid crystalline morphology and stir opalescence were produced by holding the resin at the 120°C temperature for 20 minutes. After a total of 23 minutes at 120°C, shear was applied to the resin by moving the glass coverslip across the top of the resin. As a result of the application of shear, uniaxial orientation of the liquid crystal domains was visually observable in the direction that shear was applied. After one hour at 120°C, the resin was heated at 10°C per minute to 250°C. At 250°C, the shear oriented morphology produced at 120°C was observed to be maintained. A second portion of the powder was placed between two glass plates and heated directly to 140°C. After one minute, the isotropic melt produced was cooled at 10°C per minute. On cooling, a nematic liquid crystalline morphology was observed at 84°C. Shear was applied at this temperature to the resin by moving the glass coverslip across the top of the resin. As a result of the application of shear at this temperature and degree of cure, uniaxial orientation of the liquid crystal domains was visually observable in the direction perpendicular to the direction that shear was applied. Following further cooling to 70°C the resin viscosity was increased and shear was again applied to the resin. As a result of the application of shear, uniaxial orientation of the liquid crystalline domains was visually observable in the direction that shear was applied. For the preparation of a cured casting, the remaining powder was transferred to an aluminum cup. The aluminum cup was placed in an oven which has been preheated to 140°C and the powder was observed to melt to a translucent liquid. After 5 minutes at 140°C, the oven temperature was reduced to 120°C and maintained therein for 3 hours before increasing the temperature 20°C per hour to a final temperature of 200°C. After four hours at 200°C, an opaque casting was recovered from the aluminum cup. This casting exhibited a high level of birefringence when viewed by optical microscopy (70X magnification) under crosspolarized light. Differential scanning calorimetry of a portion of the casting using the aforementioned conditions revealed a glass transition temperature of 190°C.

EXAMPLE 2

Preparation of a Cured Composition of the Diglycidyl Ester of 4,4'-Stilbenedicarboxylic Acid and 4,4'-Stilbenedicarboxylic Acid

A portion (0.3338 gram, 0.00173 epoxide equivalent) of the diglycidyl ester of 4,4'-stilbenedicarboxylic acid from Example 1-F and a portion (0.2324 gram, 0.00173 -COOH equivalent) of 4,4'-stilbenedicarboxylic acid from Example 1-E were dissolved in acetone (30 milliliters) containing tetrabutylphosphonium acetate•acetic acid complex (0.0012 gram, 0.36 phr based on the glycidyl ester reactant used). After mixing the solution for one hour, a homogeneous powder mixture was recovered by evaporation of the acetone solvent. Differential scanning calorimetry analysis of a portion (10.2 milligrams) of the powder heated at 10° C per minute under nitrogen flowing at 35 cubic centimeters per minute revealed an exotherm (199 joules per gram) between 130 and 250° C. A portion of the powder was placed between two glass plates and heated directly to 160° C at which point an opaque melt containing dispersed birefringent domains was observed via optical microscopy (70X magnification) under crosspolarized light. Following formation of the opaque melt, the resin solidified within 90 seconds to an opaque, birefringent solid. For the preparation of a cured casting, the remaining powder was transferred to an aluminum cup. The aluminum cup was placed in an oven which has been preheated to 160° C. After one hour at 160° C, the oven temperature was increased 30° C per hour to a final temperature of 230° C. After six hours at 230° C, an opaque casting was recovered from the aluminum cup. This casting exhibited a crystalline appearance when viewed by optical microscopy (70X magnification) under crosspolarized light. Differential scanning calorimetry of a portion of the casting using the aforementioned conditions revealed a glass transition temperature of 246° C.

EXAMPLE 3

A. Synthesis of 4,4'-Dicarboxychalcone

4-Carboxyacetophenone (19.70 grams, 0.12 mole), 4-carboxybenzaldehyde (18.02 grams, 0.12 mole) and absolute ethanol (500 milliliters) were added to a one liter glass resin kettle reactor and stirred as a slurry under a nitrogen flowing at one liter per minute. The slurry was cooled to 5° C using an external cooling bath, then sparging with anhydrous hydrogen chloride commenced and induced a maximum exotherm to 26° C one minute later. At this time, sparging was stopped and cooling back to 5° C completed over the next two minutes. Once the 5° C temperature was reachieved, sparging with hydrogen chloride resumed and induced a maximum exotherm to 7° C. Sparging continues until cooling reestablishes the 5° C reaction temperature and was then terminated. The thick light yellow colored, stirred slurry was allowed to warm to room temperature (24° C) over a fifteen hour period. The crude product was recovered by filtration and washed with deionized water until the wash water possessed a neutral pH. After drying in a vacuum oven at 65° C, a constant weight of 31.2 grams of a crystalline, light yellow colored powder was obtained. Nuclear magnetic resonance spectroscopy and fourier transform infrared spectrophotometric analysis of a potassium chloride pellet of the product demonstrated the presence of a minor amount of the aldol in addition to the desired 4,4'-dicarboxychalcone.

Dehydration of the residual aldol was completed via addition of a portion (19.23 grams) of the crystalline, light yellow colored powder to phosphoric acid (85 percent) (400 grams) in a one liter glass round bottom reactor. Stirring and heating commenced until the slurry reached a temperature of 150° C. After one hour at 150° C, additional phosphoric acid (200 grams) was added to the stirred slurry with cooling to 100° C. After 12 hours at the 100° C temperature, the slurry was diluted with deionized water (1000 milliliters), filtered through paper, and the resultant product was washed with deionized water until the wash water possessed a neutral pH. After drying in a vacuum oven at 100° C, a constant weight of 17.08 grams of crystalline, light yellow colored powder was obtained. Nuclear magnetic resonance spectroscopy and fourier transform infrared spectrophotometric analysis of a potassium chloride pellet of the product confirmed the product structure of dicarboxychalcone (ketone carbonyl absorbance at 1663 $cm^{-1}$, carboxylic acid carbonyl absorbance at 1689 $cm^{-1}$, carboxylic acid O-H stretching absorbances at 2993, 2884, 2825, 2671 and 2546 $cm^{-1}$). Differential scanning calorimetry of a portion (11.40 milligrams) of the product heated at 10° C per minute under nitrogen flowing at 35 cubic centimeters per minute revealed a sharp melting point endotherm at 369.3° C which was immediately followed by exothermic decomposition.

B. Epoxidation of 4,4'-Dicarboxychalcone

4,4'-Dicarboxychalcone (14.81 grams, 0.10 -COOH equivalent) from A above, epichlorohydrin (231.33 grams, 2.5 mole) and tetrabutylammonium chloride (0.148 gram, 1.0 % wt. of the diacid reactant used) were added to a one liter glass round bottom reactor and heated to 60 °C with magnetically driven stirring under a nitrogen atmosphere flowing at a rate of one liter per minute. After sixteen hours at the 60 °C reaction temperature, fourier transform infrared spectrophotometric analysis demonstrated incomplete conversion of the carboxylic acid groups (acid carbonyl absorbance at 1689 $cm^{-1}$) to ester groups (ester carbonyl absorbance at 1716 $cm^{-1}$) hence the reaction temperature was increased to 80 °C. After 167 minutes at the 80 °C reaction temperature, fourier transform infrared spectrophotometric analysis demonstrated complete conversion of the carboxylic acid groups to ester groups concurrent with the formation of a hazy, light brown colored solution. At this time, a water separator was interspersed between the reactor and the chilled (-2.5 °C) glycol condenser and an addition funnel containing sodium hydroxide (4.5 grams, 0.113 mole) dissolved in deionized water (5.5 grams, 55 % wt. of the solution) and a vacuum line were added to the reactor. The nitrogen purge was shut off simultaneous with initiation of the vacuum. The vacuum and reaction temperature were equilibrated at 84 mm Hg and 60 °C, respectively and such that a vigorous reflux was maintained with continuous return of dry epichlorohydrin from the water separator to the reactor. After equilibration, dropwise addition of the aqueous sodium hydroxide commenced accompanied by a gradual reduction in vacuum and reaction temperature. After 71 minutes, addition of the aqueous sodium hydroxide was complete and vacuum and reaction temperature were at 65 mm Hg and 55 °C, respectively. After an additional 3 hours at the 65 mm Hg vacuum and 55 °C reaction temperature, heating ceased and the product slurry was cooled to 50 °C. The recovered slurry was filtered under a nitrogen atmosphere and the resultant light amber colored solution rotary evaporated under a vacuum (1 mm Hg final conditions) at 90 °C for 30 minutes. The product was recovered as a powder. The powder product was dissolved in methylene chloride (100 milliliters), then washed with deionized water (25 milliliters). The recovered methylene chloride layer was dried over anhydrous sodium sulfate, filtered, then the resultant filtrate rotary evaporated under vacuum to a constant weight of 19.19 grams of light tan colored powder. Titration of a portion of the product revealed an epoxide equivalent weight of 218.46. Fourier transform infrared spectrophotometric analysis of a potassium chloride pellet of the product confirmed the product structure for the diglycidyl ester of 4,4'-dicarboxychalcone (ester carbonyl absorbance at 1722 $cm^{-1}$, ketone carbonyl absorbance at 1666 $cm^{-1}$, epoxide -C-O- stretching absorbance at 843 (853 slight shoulder) and 906 $cm^{-1}$).

C. Characterization of Liquid Crystallinity in the Diglycidyl Ester of 4,4'-Dicarboxychalcone

Analysis of the diglycidyl ester of 4,4'-dicarboxychalcone from B above via crosspolarized light microscopy was completed using a optical microscope equipped with a programmable hot stage using a heating rate of 10 °C per minute. The results were reported in Table III.

TABLE III

CROSSPOLARIZED LIGHT MICROSCOPY ANALYSIS OF THE DIGLYCIDYL ESTER OF 4,4'-DICARBOXYCHALCONE

| Cycle Designation | Observed Transition Temperatures (°C) | Comments |
|---|---|---|
| First heating | 30 | Birefringent crystalline solid. |
| | 95 | First fluidity noted. |
| | 106 | Isotropization completed. |
| First cooling | 71 | First crystallization noted. |
| | 30 | Birefringent semi-crystalline solid. |
| Second heating | 30 | Birefringent semi-crystalline solid. |
| | 106 | Isotropization completed. |
| Second cooling | 74 | First crystallization noted. |
| | 30 | Birefringent semi-crystalline solid. |

EXAMPLE 4

Preparation of a Cured Composition of the Diglycidyl Ester of 4,4'-Dicarboxychalcone and 4,4'-Dicarboxychalcone

A portion (2.5102 grams, 0.01149 epoxide equivalent) of the diglycidyl ester of 4,4'-dicarboxychalcone from Example 3-B and a portion (1.7024 gram, 0.01149 -COOH equivalent) of 4,4'-dicarboxychalcone from Example 3-A were dissolved in acetone (50 milliliters) containing tetrabutylphosphonium acetate•acetic acid complex (0.0088 gram, 0.35 phr based on the glycidyl ester reactant used). After mixing the solution for thirty minutes, a homogeneous powder mixture was recovered by evaporation of the acetone solvent. Differential scanning calorimetry analysis of a portion (11.4 milligrams) of the powder heated at 10 ° C per minute under nitrogen flowing at 35 cubic centimeters per minute revealed an exotherm between 110 ° C and 260 ° C. For the preparation of a cured casting, a portion (3.5 grams) of the powder was transferred to a 1 inch by 1 inch by 0.125 inch stainless steel mold. The mold was placed in a mechanical press which has been preheated to 140 ° C. Once in the press, pressure was slowly applied over a seven minute period until 10,000 psi was achieved. After two hours at 140 ° C and 10,000 psi, the press temperature was increased 180 ° C where it was maintained for four hours before cooling to room temperature (24 ° C). At room

temperature, an opaque casting was recovered from the mold and was postcured for four hours at 230°C, then four hours at 260°C. The postcured casting exhibited a high level of birefringence when viewed by optical microscopy (70X magnification) under crosspolarized light. Differential scanning calorimetry of a portion of the casting using the aforementioned conditions revealed a glass transition temperature of 218°C.

## EXAMPLE 5

### A. Synthesis of 4,4'-Dicarboxydiphenylazomethine

4-Aminobenzoic acid (20.57 grams, 0.15 mole), 4-carboxybenzaldehyde (22.52 grams, 0.15 mole) and tetrahydrofuran (600 milliliters) were added to a one liter glass resin kettle reactor and stirred under a nitrogen atmosphere flowing at one liter per minute with heating. Once the temperature reached 50°C it was held therein for the next five hours. After this time, the solution was recovered and rotary evaporated under a vacuum at 50°C until a total volume of 300 milliliters was reached. The recovered solution was cooled to room temperature (24°C) the mixed with methylene chloride (500 milliliters). The precipitated crystalline product was recovered by filtration. After drying in a vacuum oven at 90°C and 5 mm Hg, a constant weight 16.4 grams of a crystalline, brilliant yellow colored powder was obtained. Fourier transform infrared spectrophotometric analysis of a potassium chloride pellet of the product confirmed the product structure of 4,4'-dicarboxydiphenylazomethine (azomethine -C=N- absorbance contained in a complex band of peaks with minima at 1570, 1589 and 1609 $cm^{-1}$, carboxylic acid carbonyl absorbance at 1689 $cm^{-1}$, carboxylic acid O-H stretching absorbances at 2991, 2884, 2818, 2672 and 2552 $cm^{-1}$). Differential scanning calorimetry of a portion (5.90 milligrams) of the product heated at 10°C per minute under nitrogen flowing at 35 cubic centimeters per minute revealed a sharp melting point endotherm at 235.5°C which was immediately followed by exothermic decomposition.

### B. Epoxidation of 4,4'-Dicarboxydiphenylazomethine

4,4'-Dicarboxydiphenylazomethine (15.00 grams, 0.1114 -COOH equivalent from equivalent) from A above, epichlorohydrin (257.8 grams, 2.79 mole) and tetrabutylammonium chloride (0.15 gram, 1.0 % wt. of the diacid reactant used) were added to a one liter glass round bottom reactor and heated to 60°C with magnetically driven stirring under a nitrogen atmosphere flowing at a rate of one liter per minute. After seventeen hours at the 60°C reaction temperature, fourier transform infrared spectrophotometric analysis demonstrated incomplete conversion of the carboxylic acid groups (acid carbonyl absorbance at 1696 $cm^{-1}$) to ester groups (ester carbonyl absorbance at 1716 $cm^{-1}$) hence the reaction temperature was increased to 80°C. After 217 minutes at the 80°C reaction temperature, fourier transform infrared spectrophotometric analysis demonstrated complete conversion of the carboxylic acid groups to ester groups concurrent with the formation of a light yellow colored solution. At this time, a water separator was interspersed between the reactor and the chilled (-2.5°C) glycol condenser and an addition funnel containing sodium hydroxide (5.01 grams, 0.1254 mole) dissolved in deionized water (6.13 grams, 55 % wt. of the solution) and a vacuum line were added to the reactor. The nitrogen purge was shut off simultaneous with initiation of the vacuum. The vacuum and reaction temperature were equilibrated at 84 mm Hg and 60°C, respectively and such that a vigorous reflux was maintained with continuous return of dry epichlorohydrin from the water separator to the reactor. After equilibration, dropwise addition of the aqueous sodium hydroxide commenced accompanied by a gradual reduction in vacuum and reaction temperature. After 60 minutes, addition of the aqueous sodium hydroxide was complete and vacuum and reaction temperature were at 65 mm Hg and 55°C, respectively. After an additional 3 hours at the 65 mm Hg vacuum and 55°C reaction temperature, heating ceases and the product slurry was cooled to 50°C. The recovered slurry was filtered under a nitrogen atmosphere and the resultant light amber colored solution rotary evaporated under a vacuum (1 mm Hg final conditions) at 105°C for 45 minutes. The product was recovered as a powder. The powder product was dissolved in methylene chloride (100 milliliters), then washed with deionized water (25 milliliters). The recovered methylene chloride layer was dried over anhydrous sodium sulfate, filtered, then the resultant filtrate rotary evaporated under a vacuum to a constant weight of 20.84 grams of white powder. Titration of a portion of the product revealed an epoxide equivalent weight of 209.31 (corrected for background from the azomethine nitrogen). Fourier transform infrared spectrophotometric analysis of a potassium chloride pellet of the product confirmed the product structure for the diglycidyl ester of 4,4'-dicarboxydiphenylazomethine (ester carbonyl absorbance at 1716 $cm^{-1}$, azomethine -C=N-absorbance contained in a complex band of peaks with minima at 1576, 1596, 1602 (slight shoulder) and 1629 $cm^{-1}$, epoxide -C-O-stretching at 852 and 905 $cm^{-1}$).

C. Characterization of Liquid Crystallinity in the Diglycidyl Ester of 4,4'-Dicarboxydiphenylazomethine

Analysis of the diglycidyl ester of 4,4'-dicarboxydiphenylazomethine from B above via crosspolarized light microscopy was completed using a optical microscope equipped with a programmable hot stage using a heating rate of 10 ° C per minute. The results are reported in Table IV.

TABLE IV

CROSSPOLARIZED LIGHT MICROSCOPY ANALYSIS OF THE DIGLYCIDYL ESTER OF 4,4'-DICARBOXYDIPHENYLAZOMETHINE

| Cycle Designation | Observed Transition Temperatures (°C) | Comments |
|---|---|---|
| First heating | 30 | Birefringent semi-solid. |
| | 40 | First fluidity noted. |
| | 59 | Isotropization completed. |
| First cooling | 50 | First birefringent droplets observed. |
| | 44 | Batonnets first observed. |
| | 40 | First mobile mosaic texture observed. |
| | 23 | First crystallization noted after 30 minutes. |
| Second heating | 30 | Birefringent semi-solid. |
| | 82 | Isotropization completed. |
| Second cooling | 54 | First birefringent droplets observed. |
| | 30 | First mobile mosaic texture observed. |
| | 23 | First crystallization noted after 30 minutes. |

The diglycidyl ester was a monotropic liquid crystal with a smectic texture. Analysis of a portion (19.83 milligrams) of the diglycidyl ester of 4,4'-dicarboxydiphenylazomethine from B above via differential

scanning calorimetry was completed using a heating and cooling rate of 10°C per minute under nitrogen flowing at 35 cubic centimeters per minute over a temperature range of -50°C to 125°C. The results are reported in Table V.

TABLE V

DIFFERENTIAL SCANNING CALORIMETRY ANALYSIS OF THE DIGLYCIDYL ESTER OF 4,4'-DICARBOXYDIPHENYLAZOMETHINE

| Cycle Designation | Observed Transition Temperatures (°C) peak/range | Enthalpy (J/G) | Comments |
|---|---|---|---|
| First heating | -6.3/-11.6 - -0.9 | -- | Baseline inflection |
| | 59/31-97 | 36.5 | Endotherm |
| First cooling | 41/52-28 | 3.2 | Exotherm |
| | 9/14 - -2 | 0.8 | Exotherm |
| Second heating | -5.6/-9.2 - -1.9 | -- | Baseline inflection |
| | 10.3/7.0-13.5 | -- | Baseline inflection |
| | 46/29-52 | 0.7 | Endotherm |
| | 71/52-91 | 1.0 | Endotherm |
| Second cooling | 38/44-24 | 1.8 | Exotherm |
| | 8/11-0 | 0.5 | Exotherm |

EXAMPLE 6

Preparation of a Cured Composition of the Diglycidyl Ester of 4,4'-Dicarboxydiphenylazomethine and Sulfanilamide

Sulfanilamide (0.1456 gram, 0.0034 amine equivalent) was added to a portion (0.6448 grams, 0.0031 epoxide equivalent) of the diglycidyl ester of 4,4'-dicarboxydiphenylazomethine from Example 5-B as a melt contained in an aluminum cup in an oven which has been preheated to 130°C. After 10 minutes, all of the sulfanilamide has dissolved, then the oven temperature was reduced to 100°C. Differential scanning calorimetry analysis of a portion (14.4 milligrams) of the resin mixture heated at 10°C per minute under nitrogen flowing at 35 cubic centimeters per minute revealed an exotherm (332 joules per gram) between 117 and 254°C. For the preparation of a cured casting, the resin mixture was maintained at the 100°C temperature for four hours before increasing the temperature 20°C per hour to a final temperature of 160°C. After six hours at 160°C, a semi-translucent casting was recovered from the aluminum cup. The postcured casting exhibited a dispersed second phase when viewed by optical microscopy (70X magnification) under crosspolarized light. Differential scanning calorimetry of a portion of the casting using the aforementioned conditions revealed a glass transition temperature of 153°C. After postcuring this casting for twelve hours at 180°C, the glass transition was observed by differential scanning calorimetry to have increased to 209°C.

EXAMPLE 7

A. Synthesis of 4,4'-Dimethylbenzanilide

p-Methylbenzoic acid (95.31 grams, 0.70 mole), sodium ethoxide catalyst (0.2144 gram, 0.225 % wt. of the p-methylbenzoic acid used) and N,N'-dimethylacetamide (575 grams) were added to a reactor equipped with a reflux condenser and stirred under a nitrogen atmosphere at 35 ° C to provide a solution. p-Methylphenyl isocyanate (97.87 grams, 0.735 mole) was added over a two minute period inducing an exotherm to 40 ° C. At this time, heating of the reactor commenced and a 160 ° C temperature was achieved 52 minutes later. After three hours at the 160 ° C reaction temperature, the reactor was cooled to 30 ° C the the contents poured into deionized water (3.50 liters). A precipitated white crystalline product was recovered by filtration of the aqueous slurry then dried in a vacuum oven at 80 ° C and 5 mm Hg for fifteen hours. The dry product and methanol (700 milliliters) were stirred together with heating to provide a solution at 63 ° C. After cooling the methanol solution to 4 ° C for fourteen hours, a white crystalline product was filtered off and dried at 70 ° C and 5mm Hg in a vacuum oven to a constant weight of 135.6 grams. Fourier transform infrared spectrophotometric analysis of a potassium chloride pellet of the product confirmed the product structure of 4,4'-dimethylbenzanilide (amide carbonyl absorbance at 1649 $cm^{-1}$ and amide N-H stretching absorbance at 3349 and 3290 (shoulder) $cm^{-1}$.

B. Synthesis of 4,4'-Dicarboxybenzanilide

4,4'-Dimethylbenzanilide (45.05 grams, 0.40 methyl equivalent) from A above, potassium permanganate (75.0 grams, 0.475 mole) and deionized water (1250 grams) were added to a reactor equipped with a reflux condenser and stirred with heating. After 46 minutes a reflux temperature of 105 ° C was achieved and maintained. After an additional 68 minutes, all of the purple color caused by the potassium permanganate was gone, hence a second portion (37.5 grams, 0.2375 mole) of potassium permanganate was added to the slurry. After an additional 60 minutes, the purple color induced by the potassium permanganate was again gone, hence a final portion (37.5 grams, 0.2375 mole) of potassium permanganate was added to the slurry. After an additional two hours at the 105 ° C reaction temperature, heating ceases and the slurry was cooled to 50 ° C. The slurry was filtered through a pair of fritted glass funnels. The resultant clear, yellow colored filtrate was rotary evaporated under vacuum until a total volume of 800 milliliters was obtained. Concentrated hydrochloric acid (75 milliliters) was added to the stirred concentrated filtrate and the resultant precipitate was then recovered by filtration. The recovered precipitate was added to a beaker along with deionized water (750 milliliters), and the stirred contents were then brought to a boil. After cooling the aqueous slurry to 4 ° C for twelve hours, a white crystalline product was filtered off and dried at 70 ° C and 5mm Hg in a vacuum oven to a constant weight of 11.03 grams. Fourier transform infrared spectrophotometric analysis of a potassium chloride pellet of the product confirmed the product structure of 4,4'-dicarboxybenzanilide (combined amide carbonyl and carboxylic acid carbonyl absorbance at 1689 $cm^{-1}$, amide N-H stretching absorbance at 3469 and 3323 $cm^{-1}$, carboxylic acid O-H stretching absorbances at 2984, 2825, 2665 and 2546 $cm^{-1}$. Differential scanning calorimetry of a portion (10.50 milligrams) of the product heated at 10 ° C per minute under nitrogen flowing at 35 cubic centimeters per minute revealed a sharp melting point endotherm at 383.7 ° C.

C. Epoxidation of 4,4'-Dicarboxybenzanilide

4,4'-Dicarboxybenzanilide (9.98 grams, 0.070 - COOH equivalent from equivalent) from B above, epichlorohydrin (323.9 grams, 3.50 mole) and tetrabutylammonium chloride (0.1 gram, 1.0 % wt. of the diacid reactant used) were added to a one liter glass round bottom reactor and heated to 80 ° C with magnetically driven flowing at a rate of one liter per minute. After four hours at the 80 ° C reaction temperature, infrared spectrophotometric analysis demonstrated incomplete conversion of the carboxylic acid groups (acid carbonyl absorbance at 1676 $cm^{-1}$; note: amide carbonyl absorbance overlays the acid carbonyl absorbance) to ester groups (ester carbonyl absorbance at 1722 $cm^{-1}$). At this time, the reaction temperature was decreased to 60 ° C. After 241 minutes at the 60 ° C reaction temperature, fourier transform infrared spectrophotometric analysis demonstrated complete conversion of the carboxylic acid groups to ester groups concurrent with the formation of a hazy, light amber colored solution. At this time, a water separator was interspersed between the reactor and the chilled (-2.5 ° C) glycol condenser and an addition funnel containing sodium hydroxide (3.15 grams, 0.0788 mole) dissolved in deionized water (3.85 grams, 55 % wt. of the solution) and a vacuum line were added to the reactor. The nitrogen purge was shut off simultaneous with initiation of the vacuum. The vacuum and reaction temperature were equilibrated at 84 mm Hg and 60 ° C, respectively and such that a vigorous reflux was maintained with continuous return of dry epichlorohydrin from the water separator to the reactor. After equilibration, dropwise addition of the aqueous sodium hydroxide commenced accompanied by a gradual reduction in vacuum and reaction

temperature. After 60 minutes, addition of the aqueous sodium hydroxide was complete and vacuum and reaction temperature were at 65 mm Hg and 55 ° C, respectively. After an additional 2 hours at the 65 mm Hg vacuum and 55 ° C reaction temperature, heating ceases and the product slurry was cooled to 50 ° C. The recovered slurry was filtered under a nitrogen atmosphere and the resultant light amber colored solution rotary evaporated under a vacuum (2 mm Hg final conditions) at 90 ° C for 45 minutes. The product was recovered as a viscous liquid. The liquid product was dissolved in methylene chloride (100 milliliters), then washed with deionized water (25 milliliters). The recovered methylene chloride layer was dried over anhydrous sodium sulfate, filtered, then the resultant filtrate rotary evaporated under a vacuum to a constant weight of 12.42 grams of viscous, light yellow colored liquid which solidified upon standing at room temperature (24 ° C). Titration of a portion of the product revealed an epoxide equivalent weight of 207.64. Fourier transform infrared spectrophotometric analysis of a neat film of the product on a potassium chloride plate confirmed the product structure for the diglycidyl ester of 4,4'-dicarboxybenzanilide (ester carbonyl absorbance at 1722 $cm^{-1}$, amide carbonyl absorbance at 1682 $cm^{-1}$, amide N-H stretching absorbance at 3449 and 3363 $cm^{-1}$, epoxide -C-O- stretching at 846 (852 slight shoulder) and 905 $cm^{-1}$).

D. Characterization of Liquid Crystallinity in the Diglycidyl Ester of 4,4'-Dicarboxybenzanilide

Analysis of the diglycidyl ester of 4,4'-dicarboxybenzanilide from C above via crosspolarized light microscopy was completed using a optical microscope equipped with a programmable hot stage using a heating and cooling rate of 10 ° C per minute. The results are reported in Table V.

TABLE V

CROSSPOLARIZED LIGHT MICROSCOPY ANALYSIS OF THE DIGLYCIDYL ESTER OF 4,4'-DICARBOXYBENZANILIDE

| Cycle Designation | Observed Transition Temperatures (°C) | Comments |
|---|---|---|
| First heating | 30 | Birefringent semi-solid. |
| | 40 | First fluidity noted. |
| | 47 | Dispersed birefringent domains and opalescence observed. |
| | 84 | Isotropization. |
| First cooling | 9 | Opacity and birefringent morphology observed in semi-solid resin. |
| Second heating | 27 | Fluidity and dispersed birefringent domains first observed. |
| | 55 | Birefringent domains increase in number. |
| | 81 | Isotropization. |
| Second cooling | 9 | Opacity and birefringent morphology observed in semi-solid resin. Crystallization noted after five minutes. |

EXAMPLE 8

A. Preparation of 4,4'-Dihydroxybenzophenone Oxime from 4,4'-Dihydroxybenzophenone

4,4'-Dihydroxybenzophenone (100.0 grams, 0.467 mole) was added to ethanol (300 milliliters) in a one liter Erlenmeyer flask and stirred. Once the 4,4'-dihydroxybenzophenone was in solution, a solution of

hydroxylamine hydrochloride (48.6 grams, 0.699 mole) and sodium acetate (57.4 grams, 0.70 mole) in water (70 milliliters) was added to the flask, followed by additional ethanol (100 milliliters). The stirred mixture was heated on a hot plate to a gentle reflux (75°C). After four hours at reflux, the stirred solution was cooled to room temperature and then filtered. The resultant filter cake was washed with ethanol (100 milliliters), then the total filtrate obtained (600.4 grams) concentrated to a weight of 219.2 grams by evaporation of part of the ethanol. The concentrated solution and deionized water (600 milliliters) were placed in a one liter Erlenmeyer flask and stirred. The addition of the deionized water induced the formation of a white precipitate. After thirty minutes of stirring, the slurry was filtered and the recovered white powder was dried in a vacuum oven to a constant weight of 98.22 grams. Fourier transform infrared spectrophotometric analysis of a potassium bromide pellet of the product confirmed the product structure for 4,4'-dicarboxybenzophenone oxime (hydoxyl O-H stretching at 3400 $cm^{-1}$, aromatic -C-O- stretching at 1235 $cm^{-1}$, aromatic ring -C-C- stretching at 1607 and 1513 $cm^{-1}$). Differential scanning calorimetry of a portion of the product heated at 10°C per minute under nitrogen flowing at 35 cubic centimeters per minute revealed a melting point endotherm at 155°C followed by an exotherm (rearrangement of 4,4'-dihydroxybenzophenone oxime to 4,4'-dihydroxybenzanilide) at 155 to 188°C. Following this exotherm, a melting point endotherm for the rearrangement product was observed at 269°C. Liquid chromatographic analysis of a portion of the 4,4'-dihydroxybenzophenone product indicates a purity of 97.8%.

B. Preparation of 4,4'-Dihydroxybenzanilide from 4,4'-Dihydroxybenzophenone Oxime

4,4'-Dihydroxybenzophenone oxime (66.0 grams, 0.288 mole) from A above and acetic acid (330 milliliters) were added to a 500 milliliter round bottom flask equipped with a stirrer, nitrogen purge, water cooled condenser and thermostatically controlled heating mantle. p-Toluenesulfonic acid catalyst (1.85 grams, 0.027 mole) was added to the stirred reaction mixture, and heating commenced. After heating for two hours at 83°C, a precipitate formed. The reaction mixture was then stirred for an additional two hours at 87°C and then diluted with deionized water (25 milliliters). Thirty minutes later, the contents of the reaction flask were transferred to a one liter Erlenmeyer flask and stirred. Immediately following this transfer, additional deionized water (400 milliliters) was added. The mixture was stirred for an additional 45 minutes, then filtered. The filter cake obtained was washed with deionized water (800 milliliters), then recovered and dried in a vacuum oven to a constant weight of 54.2 grams of light beige colored product. Fourier transform infrared spectrophotometric analysis of a potassium bromide pellet of the product confirmed the product structure for 4,4'-dicarboxybenzanilide (amide N-H stretching absorbance at 3322 $cm^{-1}$, aromatic -C-O- stretching at 1251 $cm^{-1}$, aromatic ring -C-C- stretching at 1609 and 1514 $cm^{-1}$, amide carbonyl absorbance at 1646 $cm^{-1}$). Differential scanning calorimetry of a portion of the product heated at 10°C per minute under nitrogen flowing at 35 cubic centimeters per minute revealed a sharp melting point endotherm at 273°C.

C. Preparation of an Advanced Resin Composition of the Diglycidyl Ester of 4,4'-Dicarboxybenzanilide With 4,4'-Dihydroxybenzanilide

A portion (1.0479 grams, 0.00505 epoxide equivalent) of the diglycidyl ester of 4,4'-dicarboxybenzanilide from Example 7-C and a portion (0.2314 gram, 0.00202 -OH equivalent) of 4,4'-dihydroxybenzanilide from B above were dissolved in acetone (50 milliliters) containing tetrabutylphosphonium acetate•acetic acid complex (0.0036 gram, 0.34 phr based on the glycidyl ester reactant used). After mixing the solution for ten minutes, a homogeneous semi-solid mixture was recovered by evaporation of the acetone solvent. Differential scanning calorimetry analysis of a portion (29.4 milligrams) of the powder heated at 10°C per minute under nitrogen flowing at 35 cubic centimeters per minute revealed an exotherm (156 joules per gram) between 100°C and 230°C. A portion of the mixture was placed between two glass plates and heated directly to 100°C at which point, an isotropic melt containing dispersed crystals was observed via optical microscopy (70X magnification) under crosspolarized light. After 2 hours at 100°C, the crystals present in the mixture cleared and a mobile, birefringent phase was observed. For the preparation of an advanced resin composition, the remaining mixture was transferred to an aluminum cup. The aluminum cup was placed in an oven which has been preheated to 100°C. After 2 hours at 100°C, the oven temperature was increased to 150°C and maintained therein for 4 hours prior to cooling to room temperature (22°C). After cooling to room temperature, an opaque solid was recovered from the alumninum cup. This solid exhibited a high level of phase birefringence when viewed by optical microscopy (70X magnification) under crosspolarized light. Differential scanning calorimetry of a portion of the solid using the aforementioned conditions revealed a pair of transition temperatures at 106 and 200°C.

EXAMPLE 9

Preparation of a Cured Composition of the Diglycidyl Ester of 4,4'-Dicarboxybenzanilide Cured With 4,4'-Diaminobenzanilide

4,4'-Diaminobenzanilide (0.3319 gram, 0.00548 amine equivalent) was added to a portion (1.2128 grams, 0.00548 epoxide equivalent) of the diglycidyl ester of 4,4'-dicarboxybenzanilide from Example 7-C as a melt contained in an aluminum cup in an oven which has been preheated to 100° C. This mixture was periodically stirred over the next thirty minutes. After 12 hours at 100° C, the oven temperature was increased 20° C per hour to a final temperature to 200° C. After six hours at 200° C, Differential scanning calorimetry analysis of a portion (14.4 milligrams) of the resin mixture heated at 10° C per minute under nitrogen flowing at 35 cubic centimeters per minute revealed an exotherm (332 joules per gram) between 117° C and 254° C. For the preparation of a cured casting, the resin mixture was maintained at the 100° C temperature for four hours before increasing the temperature 20° C per hour to a final temperature of 160° C. After six hours at 160° C, the oven was cooled to room temperature (22° C) and a semi-translucent casting was recovered from the aluminum cup. The postcured casting exhibited dispersed birefringent regions having a liquid crystal type texture when viewed by optical microscopy (70X and 300X magnifications) under crosspolarized light. Differential scanning calorimetry of a portion (20.0 milligrams) of the casting using the aforementioned conditions revealed a glass transition temperature of 176° C.

EXAMPLE 10

Preparation of a Cured Composition of the Diglycidyl Ester of 4,4'-Dicarboxybenzanilide Cured With 4,4'-Diaminobenzanilide

4,4'-Diaminobenzanilide (0.1731 gram, 0.00305 amine equivalent) was added to a portion (1.0542 grams, 0.00508 epoxide equivalent) of the diglycidyl ester of 4,4'-dicarboxybenzanilide from Example 7-C as a melt contained in an aluminum cup in an oven which has been preheated to 100° C. This mixture was periodically stirred over the next thirty minutes. After 12 hours at 100° C, the oven temperature was increased 20° C per hour to a final temperature to 200° C. After six hours at 200° C, the oven was cooled to room temperature (22° C) and a semi-translucent casting was recovered from the aluminum cup. The postcured casting exhibited a low level of birefringence when viewed by optical microscopy (70X magnifications) under crosspolarized light. Differential scanning calorimetry analysis of a portion (20.0 milligrams) of the casting heated at 10° C per minute under nitrogen flowing at 35 cubic centimeters per minute revealed a glass transition temperature of 180° C.

EXAMPLE 11

Preparation of an Injection Molded Casting of The Diglycidyl Ester of 4,4'-Stilbenedicarboxylic Acid Cured with Sulfanilamide

A diglycidyl ester of 4,4'-stilbenedicarboxylic acid (5.0121 grams, 0.0260 epoxide equivalents) prepared using the method of Example 1-F and having an epoxide equivalent weight of 192.7 was placed in an oven preheated to 150° C. Twenty minutes later, after melting had occurred, the oven temperature was reduced to 140 ■C. Once a 140° C temperature was achieved, sulfanilamide (1.1199 grams, 0.0260 N-H equivalents) was added and thoroughly mixed therein. After all the sulfanilamide had dissolved (3 minutes), the resinous mixture was poured into the reservoir of an injection molder preheated to 140° C. After 2 minutes, the temperature of the reservoir was reduced to 138° C and then the resin was injected through a 0.02 inch (.508 mm) by 0.375 inch (9.52 mm) rectangular flow gate into a mold preheated to 80° C and having the following dimensions: 3.0 inches x 0.125 inches (76.2 mm x 12.7 mm x 3.17 mm). The filled mold was then immediately transferred to an oven preheated to 80 ■C. After transferring to the oven, the following cure schedule was used for the resin before cooling to room temperature (23° C): 80° C for 7 hours, 90° C for 14 hours, 100° C for 6 hours, 120° C for 1 hour, 140° C for 1 hour, 160° C for 1 hour and 180° C for 12 hours. On cooling from 180° C to room temperature, the casting was removed from the mold. The glass transition temperature of the casting was measured by differential scanning calorimetry and was found to be 185° C . Following differential scanning calorimetry, the casting was tested for flexural properties. The flexural strength and modulus obtained in the resin flow direction into the mold were 15,900 psi (115 MPascals) and 410 ksi (2.8 GPascals), respectively. Thermal mechanical analyses were also performed for the casting in

the x, y and z directions from 35 °C to 180 °C at a heating rate of 10 °C per minute. The mean linear coefficients of thermal expansion obtained from these analyses were as follows: 9 ppm/°C (z coordinate/surface of casting), 69 ppm/°C (y coordinate/ flow direction). The much lower mean linear coefficient of thermal expansion in the z direction demonstrates the anisotropy which can be produced by processing methods that induce shear, such as injection molding.

## Claims

**1.** A polyglycidyl ester composition containing one or more mesogenic moieties represented by the following Formula I

Formula I

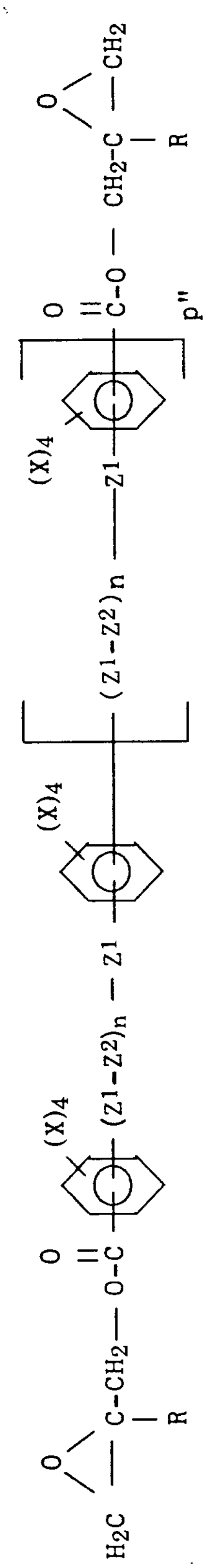

wherein at least 80 percent of the $-(Z^1-Z^2)_n-Z^1-$linkages and the glycidyl ester groups are in the para position with respect to each other; each R and $R^1$ is independently hydrogen or an aliphatic hydrocarbon group having from 1 to 4 carbon atoms; each X is independently hydrogen, a hydrocarbyl or hydrocarbyloxy group having suitably from 1 to 12 carbon atoms, a halogen atom, $-NO_2$, or $-C{\equiv}N$; each $Z^1$ is independently a direct single bond, $-CR^1=CR^1-$, $-CR^1=CR^1-CR^1=CR^1-$, $-CR^1=N-N=CR^1-$,

$-CR^1=CR^1-CO-O-(CHR^1)_{p'}-$, $-CR^1=CR^1-O-CO-(CHR^1)_{p'}-$, $-(CHR^1)_{p'}-O-CO-CR^1=CR^1-$, $-(CHR^1)_{p'}-CO-O-CR^1=CR^1-$, $-CR^1=CR^1-CO-O-$, $-O-CO-CR^1=CR^1-$, $-CO-NR^1-$, $-NR^1-CO-$, $-CO-NR^1-NR^1-CO-$, $-C\equiv C-$, $-C\equiv C-C\equiv C-$, -CO-S-, -S-CO-, $-CR^1=N-$, $-N=CR^1-$, -O-CO-, -CO-O-, $-CR^1=CR^1-CO-$, $-CO-CR^1=CR^1-$, $-CR^1=CR^1-O-CO-$, $-CO-O-CR^1=CR^1-$, $-CH_2-CH_2-CO-O-$, $-O-CO-CH_2-CH_2-$, -N=N-,

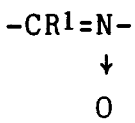 , 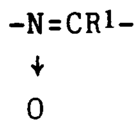 , 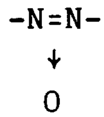 , 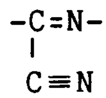 , 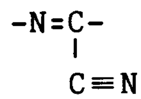 ,

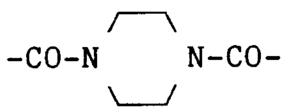 , 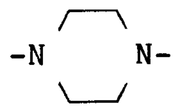 , 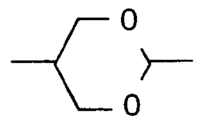 ,

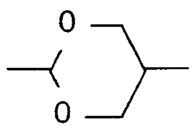 , 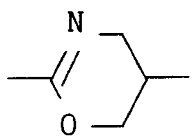 , 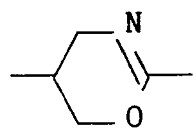 ,

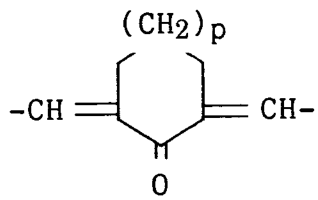 (p=0, 1, 2) ,

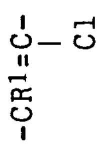
,
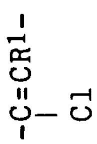
,
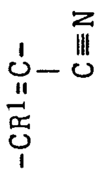
,
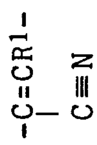
,

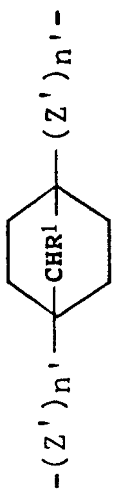 ,

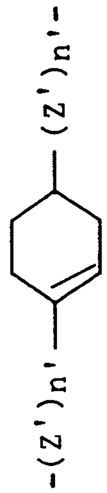 ,

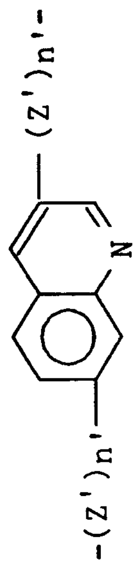 ,

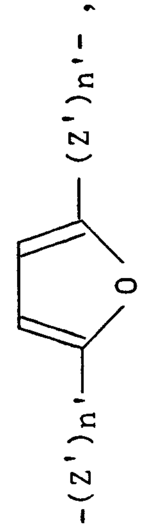 ,

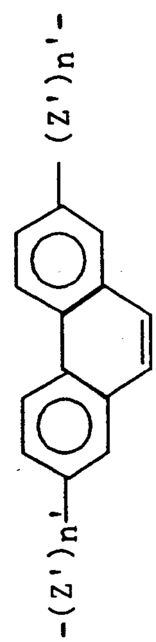 ,

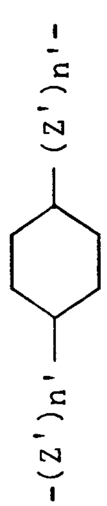 ,

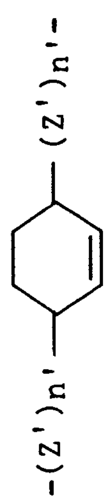 ,

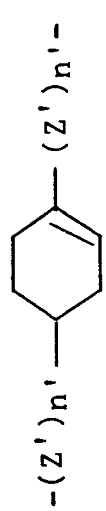 ,

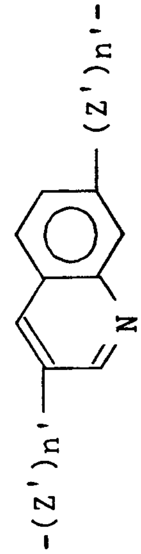 ,

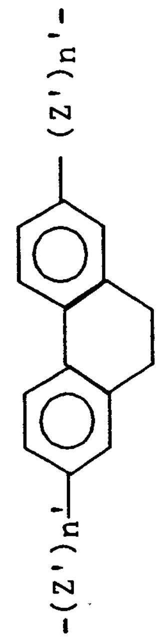 ,

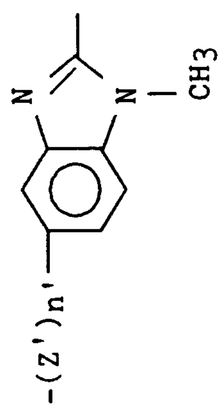,
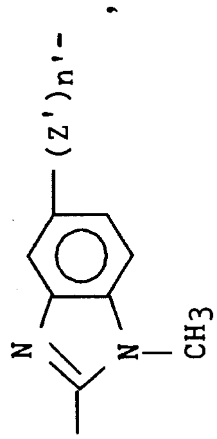,
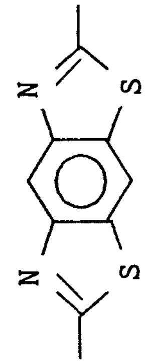,
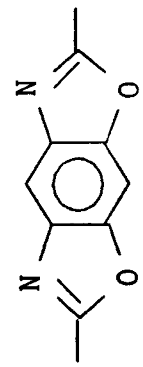,
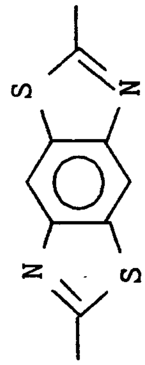;
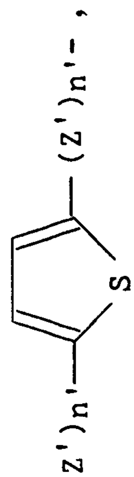,
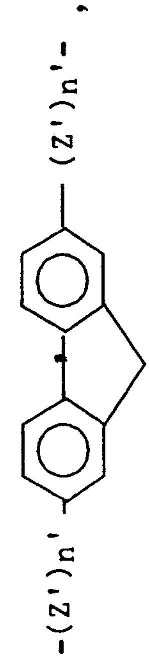,
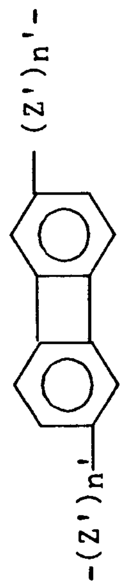,
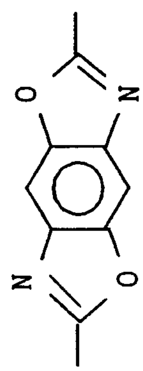

$Z^2$ is a group represented by a cyclic or bicyclic ring system containing from 5 to 12 carbon atoms and may be cycloaliphatic, polycycloaliphatic, aromatic or a combination thereof; n is 0 to 2; p' is 1 or 2; p'' has a value of zero to 100; each Z' is independently a -CO-, -O-CO-, -CO-O-, $-CO-NR^1-$, or $-NR^1-CO-$ group and each n' independently has a value of zero or one; with the proviso that the polyglycidyl ester of Formula I is not the polyglycidyl ester of 4,4'-dicarboxystilbene (R = H, X = H, n = O, p'' = O, $Z^1$ =

$-CR^1=CR^1-$ wherein both $R^1$ groups are H) or the polyglycidyl ester or polymethylglycidyl ester of bis-(4'-carboxyphenyl)-1,4-benzenediimine (R = H or CH3, X = H, n = 1 p'' = O, $Z^1$ = $-CR^1=N-$ wherein $R^1$ is H, and $Z^2$ is

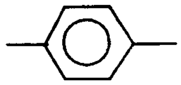 ).

2. A polyglycidyl ester composition containing one or more mesogenic moieties represented by the following Formula II

Formula II

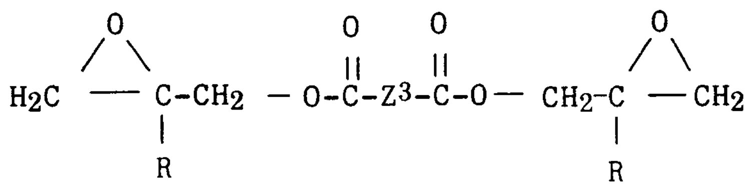

wherein $Z^3$ is

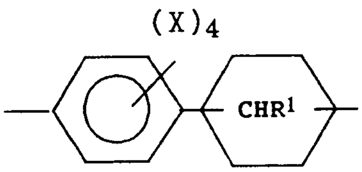 ,

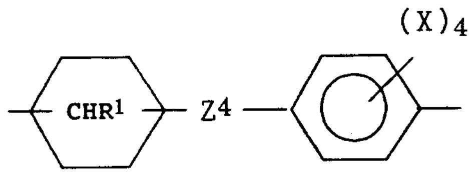 ,

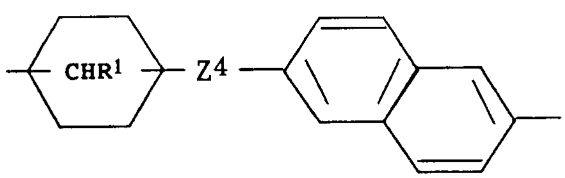 ,

or

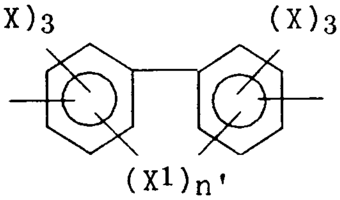 ;

and $Z^4$ is -CO-O-, -O-CO-, $-NR^1-CO-$ or $-CO-NR^1-$; and $X^1$ is a hydrocarbyl group having from 1 to 10, carbon atoms which can contain one or more heteroatoms selected from N, O, S and the like and may be saturated or unsaturated; each R and $R^1$ is independently hydrogen or an aliphatic hydrocarbon group having from 1 to 4 carbon atoms; each X is independently hydrogen, a hydrocarbyl or hydrocarbyloxy group having from 1 to 12 carbon atoms, a halogen atom, $-NO_2$, or -C≡N; and each n' is independently zero or one.

**3.** A polyglycidyl ester composition comprising a diglycidyl ester of 4,4'-dicarboxydiphenylazomethine, a diglycidyl ester of 4,4'-dicarboxybenzanilide, a diglycidyl ester of 4,4'-dicarboxychalcone, or any combination thereof.

**4.** An advanced polyglycidyl ester composition prepared by reacting

(A) one or more polyglycidyl esters containing one or more mesogenic moieties, said polyglycidyl esters being those represented by either Formula I, with the proviso that the polyglycidyl ester of Formula I may include the polyglycidyl ester of 4,4'-dicarboxystilbene or the polyglycidyl ester or polymethylglycidyl ester of bis(4'-carboxyphenyl)-1,4-benzenediimine; or Formula II; with

(B) at least one compound having an average of more than one active hydrogen atom per molecule; wherein components (A) and (B) are employed in quantities which provide a ratio of active hydrogen atoms per epoxide group of from 0.01:1 to 0.95:1.

**5.** An advanced polyglycidyl ester composition of Claim 8 wherein

(a) when component (A) is a polyglycidyl ester represented by Formula I, in which X is a hydrocarbyl or hydrocarbyloxy group, it has from 1 to 6 carbon atoms and when it is a halogen atom, it is chlorine or bromine;

(b) when component (A) is a polyglycidyl ester represented by Formula II, in which X is a hydrocarbyl or hydrocarbyloxy group, it has from 1 to 6 carbon atoms and when it is a halogen atom, it is chlorine or bromine;

(c) component (B) is a compound represented by the following Formulas XVII and XVIII

Formula XVII

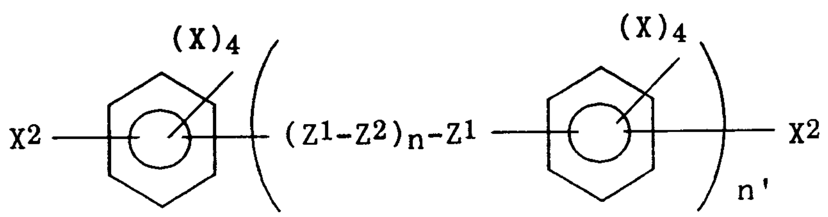

Formula XVIII

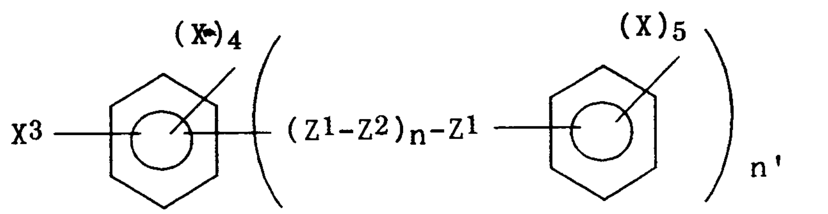

wherein $X^2$ is independently a hydroxyl, carboxylic acid, -SH, or $-NHR^2$ group; $R^2$ is an alkyl group having from 1 to 4 carbon atoms; $X^3$ is $NH_2$, $NH_2-SO_2-$, $NH_2-CO-$, or $NH_2-Z^5-O-$; $Z^5$ is an alkyl or cycloalkyl group having from 1 to 12 carbon atoms; $Z^1$ can independently be a divalent hydrocarbyl group having from 1 to 10 carbon atoms, -O-, -CO-, -SO-, $-SO_2-$, -S-, -S-S-, $-CR^1=CR^1-$, $-CR^1=CR^1-CR^1=CR^1-$, $-CR^1=N-N=CR^1-$, $-CR^1=CR^1-CO-O-(CHR^1)_{p'}-$, $-CR^1=CR^1-O-CO-(CHR^1)_{p'}-$, $-(CHR^1)_{p'}-O-CO-CR^1=CR^1-$, $-(CHR^1)_{p'}-CO-O-CR^1=CR^1-$, $-CR^1=CR^1-CO-O-$, $-O-CO-CR^1=CR^1-$, $-CO-NR^1-$, $-NR^1-CO-$, $-CO-NR^1-NR^1-CO-$, -C≡C-, -C≡C-C≡C-,-CO-S-, -S-CO-, $-CR^1=N-$, $-N=CR^1-$, -O-CO-, -CO-O-, $-CR^1=CR^1-CO-$, $-CO-CR^1=CR^1-$, $-CR^1=CR^1-O-CO-$, $-CO-O-CR^1=CR^1-$, $-CH_2-CH_2-CO-O-$, $-O-CO-CH_2-CH_2-$, -N=N-, a direct single bond,

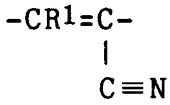 , 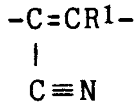 , 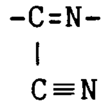 , 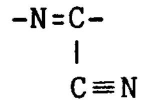 ,

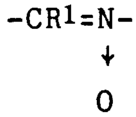 , 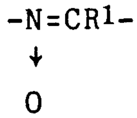 , 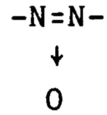 ,

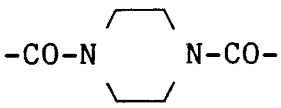 , 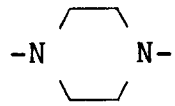 , 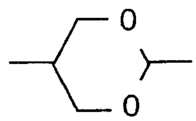 ,

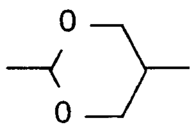 , 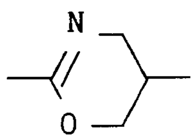 , 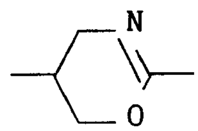 ,

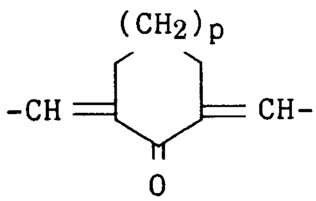 (p=0, 1, 2) ,

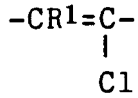 , 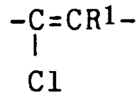 ,

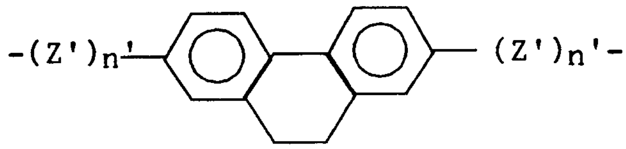 ,

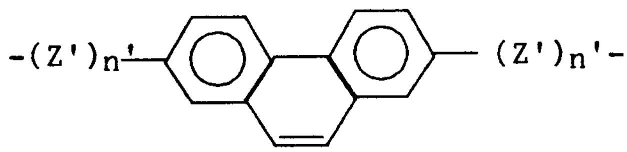 ,

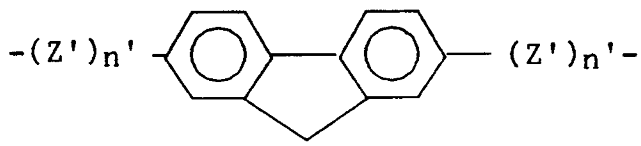 ,

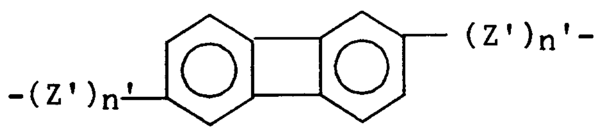 ,

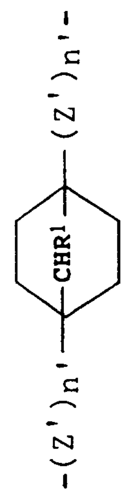 ,
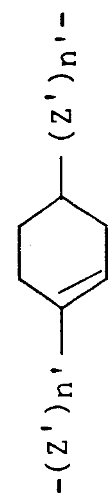 ,
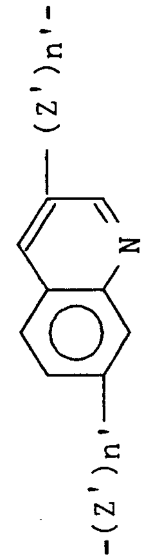 ,
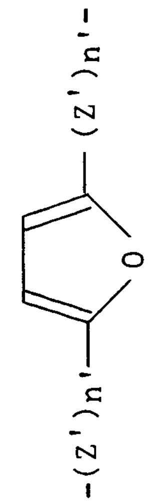 ,

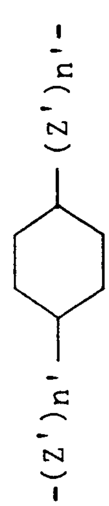 ,
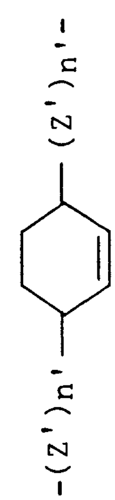 ,
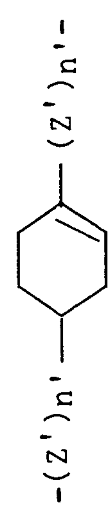 ,
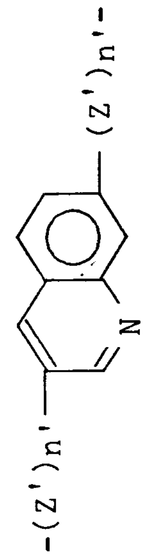 ,

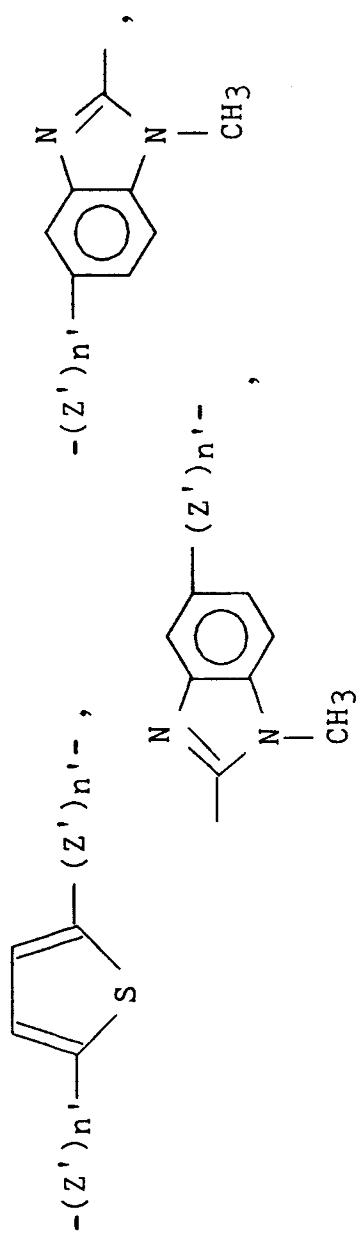

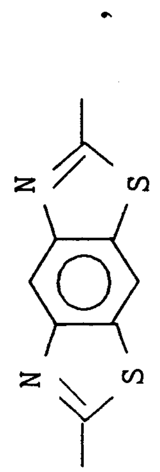

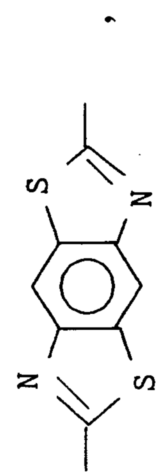

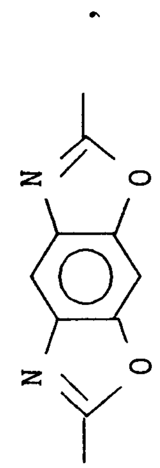

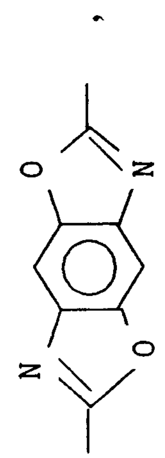

and each X is independently hydrogen, a hydrocarbyl or hydrocarbyloxy group having from 1 to 12 carbon atoms, a halogen atom, $-NO_2$ or -C≡N; $Z^2$ is a group represented by a cyclic or bicyclic ring system containing from 5 to 12 carbon atoms and may be cycloaliphatic, polycycloaliphatic, aromatic or a combination thereof; n is zero to 2; each Z' is independently a -CO-, -O-CO-, -CO-O-, $-CO-NR^1-$, or $-NR^1-CO-$ group; each $R^1$ is independently hydrogen or an aliphatic hydrocarbon group having from 1 to 4 carbon atoms and each n' independently has a value of zero or one; or any combination of any two or more compounds represented by the aforementioned Formulas XVII and XVIII; and

(d) components (A) and (B) are employed in amounts which provide a ratio of active hydrogen atoms per epoxide group of from 0.05:1 to 0.8:1.

**6.** An advanced polyglycidyl ester of Claim 4 wherein

(a) component (B) is hydroquinone, bisphenol A, 4,4'-dihydroxydiphenylmethane, 4,4'-thiodiphenol, 4,4'-sulfonyldiphenol, 4,4'-dihydroxydiphenyl oxide, 4,4'-dihydroxybenzophenone, 1,1-bis(4-hydroxyphenyl)-1-phenylethane, 3,3',5,5'-tetrachlorobisphenol A, 3,3'-dimethoxybisphenol A, 4,4'-dihydroxybiphenyl, 4,4'-dihydroxy-α,α'-diethylstilbene, 4,4'-dihydroxy-α-methylstilbene, 4,4'-dihydroxybenzanilide, 4,4'-dihydroxy-2,2'-dimethylazoxybenzene, 4,4'-dihydroxy-α-cyanostilbene, bis(4-hydroxyphenyl)terephthalate, N,N'-bis(4-hydroxyphenyl)terephthalamide, bis(4'-hydroxybiphenyl)-

terephthalate, 4,4'-dihydroxyphenylbenzoate, bis(4'-hydroxyphenyl)-1,4-benzenediimine, 4,4"-dihydroxybiphenylbenzoate, 1,4-bis(4'-hydroxyphenyl-1'-carboxamide)benzene, 1,4-bis(4'-hydroxyphenyl-1'-carboxy)benzene, 4,4'-bis(4"-hydroxyphenyl-1"-carboxy)biphenyl, terephthalic acid, 4,4'-benzanilide dicarboxylic acid, 4,4'-phenylbenzoate dicarboxylic acid, 4,4'-stilbenedicarboxylic acid, 4,4'-dicarboxybiphenyl, 4,4'-dicarboxydiphenylazomethine, aniline, 4'-sulfonamido-N-phenylbenzamide, 4'-sulfonamido-N'-phenyl-4-chlorobenzamide, 4-amino-1-phenylbenzoate, 4-amino-N-phenylbenzamide, N-phenyl-4-aminophenyl-1-carboxamide, phenyl-4-aminobenzoate, biphenyl-4-aminobenzoate, 1-phenyl-4'-aminophenylterephthalate and mixtures thereof; and
(b) components (A) and (B) are employed in amounts which provide a ratio of active hydrogen atoms per epoxide group of from 0.1:1 to 0.5:1.

**7.** An advanced polyglycidyl ester of Claim 5 wherein
(a) component (B) is hydroquinone, bisphenol A, 4,4'-dihydroxydiphenylmethane, 4,4'-thiodiphenol, 4,4'-sulfonyldiphenol, 4,4'-dihydroxydiphenyl oxide, 4,4'-dihydroxybenzophenone, 1,1-bis(4-hydroxyphenyl)-1-phenylethane, 3,3',5,5'-tetrachlorobisphenol A, 3,3'-dimethoxybisphenol A, 4,4'-dihydroxybiphenyl, 4,4'-dihydroxy-α,α'-diethylstilbene, 4,4'-dihydroxy-α-methylstilbene, 4,4'-dihydroxybenzanilide, 4,4'-dihydroxy-2,2'-dimethylazoxybenzene, 4,4'-dihydroxy-α-cyanostilbene, bis(4-hydroxyphenyl)terephthalate, N,N'-bis(4-hydroxyphenyl)terephthalamide, bis(4'-hydroxybiphenyl)-terephthalate, 4,4'-dihydroxyphenylbenzoate, bis(4'-hydroxyphenyl)-1,4-benzenediimine, 4,4"-dihydroxybiphenylbenzoate, 1,4-bis(4'-hydroxyphenyl-1'-carboxamide)benzene, 1,4-bis(4'-hydroxyphenyl-1'-carboxy)benzene, 4,4'-bis(4"-hydroxyphenyl-1"-carboxy)biphenyl, terephthalic acid, 4,4'-benzanilide dicarboxylic acid, 4,4'-phenylbenzoate dicarboxylic acid, 4,4'-stilbenedicarboxylic acid, 4,4'-dicarboxybiphenyl, 4,4'-dicarboxydiphenylazomethine, aniline, 4'-sulfonamido-N-phenylbenzamide, 4'-sulfonamido-N'-phenyl-4-chlorobenzamide, 4-amino-1-phenylbenzoate, 4-amino-N-phenylbenzamide, N-phenyl-4-aminophenyl-1-carboxamide, phenyl-4-aminobenzoate, biphenyl-4-aminobenzoate, 1-phenyl-4'-aminophenylterephthalate and mixtures thereof; and
(b) components (A) and (B) are employed in amounts which provide a ratio of active hydrogen atoms per epoxide group of from 0.1:1 to 0.5:1.

**8.** A monoglycidyl ester compound containing one or more mesogenic moieties represented by the following Formula III

Formula III

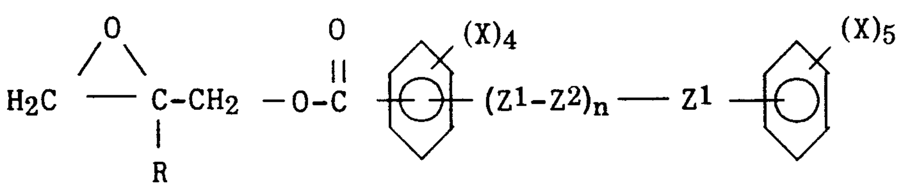

wherein at least 80 percent of the $-(Z^1-Z^2)_n-Z^1$-linkages and the glycidyl ester groups are in the para position with respect to each other; each R and $R^1$ is independently hydrogen or an aliphatic hydrocarbon group having from 1 to 4 carbon atoms; each X is independently hydrogen, a hydrocarbyl or hydrocarbyloxy group having suitably from 1 to 12 carbon atoms, a halogen atom, $-NO_2$, or -C≡N; each $Z^1$ is independently a direct single bond, $-CR^1=CR^1-$, $-CR^1=CR^1-CR^1=CR^1-$, $-CR^1=N-N=CR^1-$, $-CR^1=CR^1-CO-O-(CHR^1)_{p'}-$, $-CR^1=CR^1-O-CO-(CHR^1)_{p'}-$, $-(CHR^1)_{p'}-O-CO-CR^1=CR^1-$, $-(CHR^1)_{p'}-CO-O-CR^1=CR^1-$, $-CR^1=CR^1-CO-O-$, $-O-CO-CR^1=CR^1-$, $-CO-NR^1-$, $-NR^1-CO-$, $-CO-NR^1-NR^1-CO-$, -C≡C-, -C≡C-C≡C-, -CO-S-, -S-CO-, $-CR^1=N-$, $-N=CR^1-$, -O-CO-, -CO-O-, $-CR^1=CR^1-CO-$, $-CO-CR^1=CR^1-$, $-CR^1=CR^1-O-CO-$, $-CO-O-CR^1=CR^1-$, $-CH_2-CH_2-CO-O-$, $-O-CO-CH_2-CH_2-$, -N=N-,

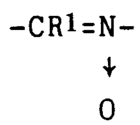 , 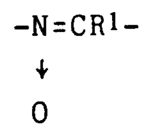 , 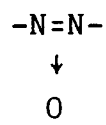 , 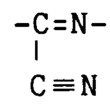 , 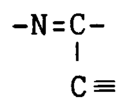 ,

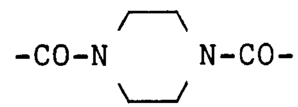 , 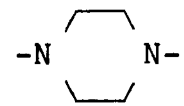 , 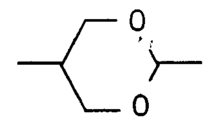 ,

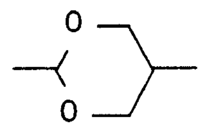 , 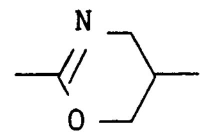 , 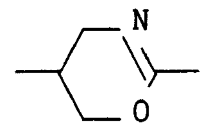 ,

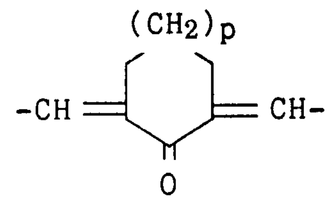 (p=0, 1, 2) ,

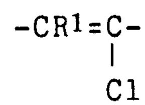 , 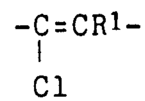 , 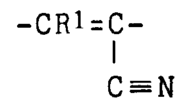 , 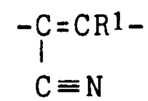 ,

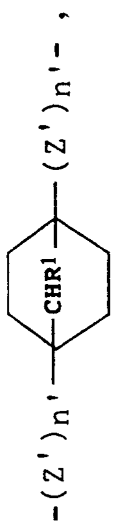 ,
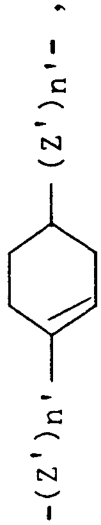 ,
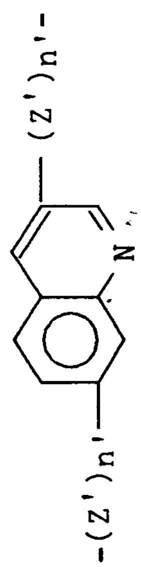 ,
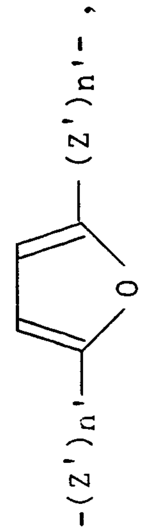 ,
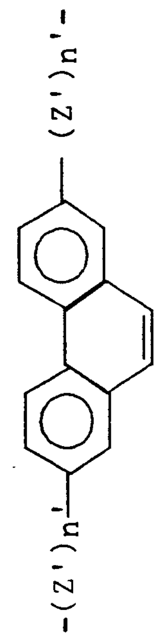 ,

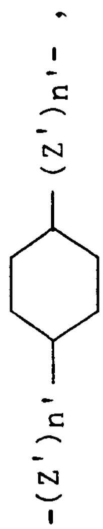 ,
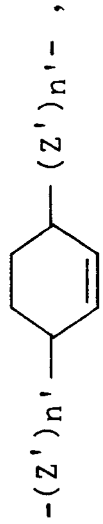 ,
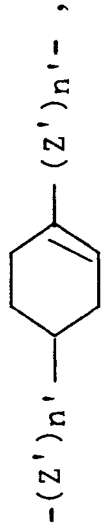 ,
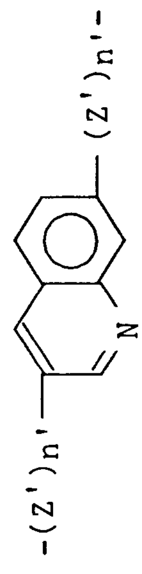 ,
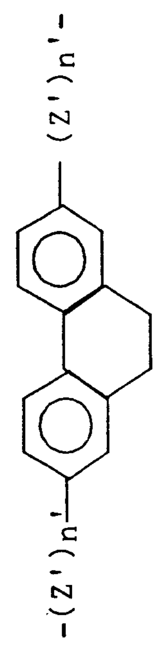 ,

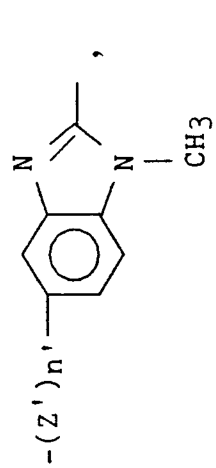 ,

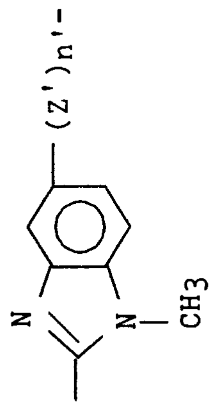 ,

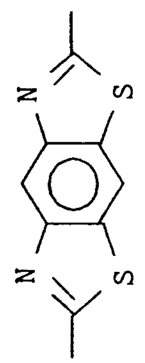 ,

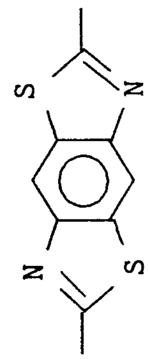 ;

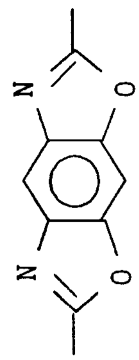 ,

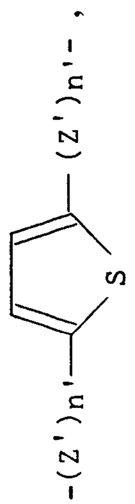 ,

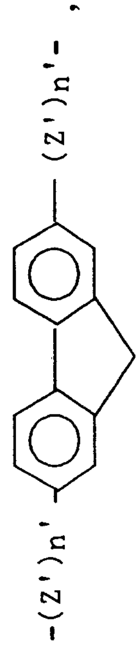 ,

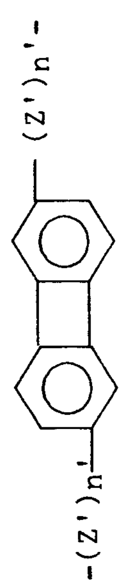 ,

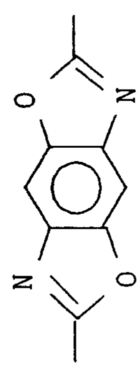 ,

$Z^2$ is a group represented by a cyclic or bicyclic ring system containing from 5 to 12 carbon atoms and may be cycloaliphatic, polycycloaliphatic, aromatic or a combination thereof; n is 0 to 2; p' is 1 or 2; each Z' is independently a -CO-, -O-CO-, -CO-O-, -CO-$NR^1$-, or -$NR^1$-CO- group and each n' independently has a value of zero or one; with the proviso that the monoglycidyl ester of Formula III is not the monoglycidyl ester of 4-carboxybiphenyl (R = H, X = H, n = O, $Z^1$ = a direct single bond).

**9.** A monoglycidyl ester compound containing one or more mesogenic moieties represented by the following Formula IV

Formula IV

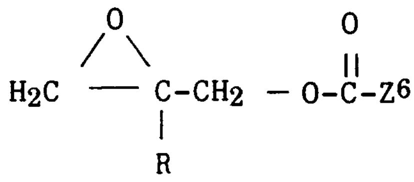

wherein $Z^6$ is

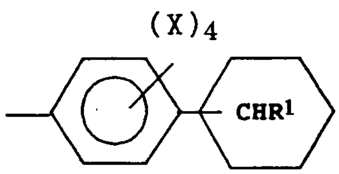
,
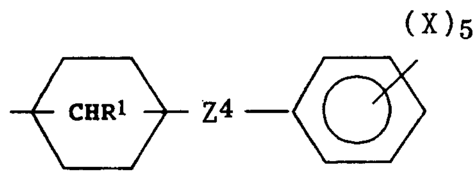
,

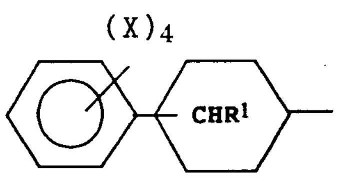
,
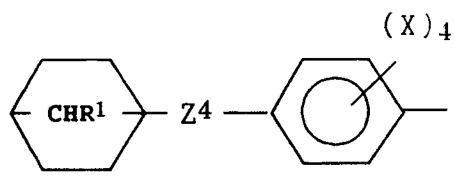
,

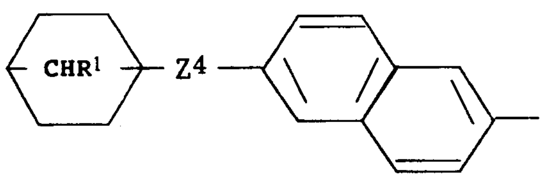
,

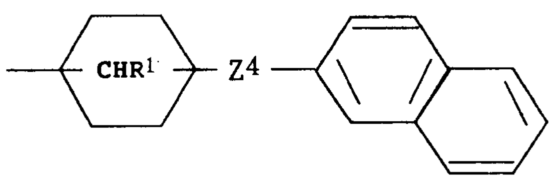
,

or

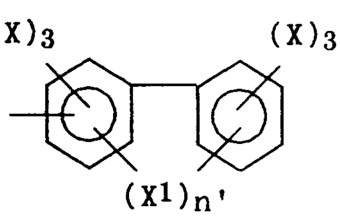
;

and $Z^4$ is -CO-O-, -O-CO-, -$NR^1$-CO- or -CO-$NR^1$-; and $X^1$ is a hydrocarbyl group having from 1 to 10,

carbon atoms which can contain one or more heteroatoms selected from N, O, S and the like and may be saturated or unsaturated; each R and $R^1$ is independently hydrogen or an aliphatic hydrocarbon group having from 1 to 4 carbon atoms; each X is independently hydrogen, a hydrocarbyl or hydrocarbyloxy group having from 1 to 12 carbon atoms, a halogen atom, $-NO_2$, or -C≡N; and each n' is independently zero or one.

**10.** A blend comprising

(A) one or more polyglycidyl esters or monoglycidyl ester compounds containing one or more mesogenic moieties represented by either Formula I with the proviso that the polyglycidyl ester of Formula I may include the polyglycidyl ester of 4,4'-dicarboxystilbene or the polyglycidyl ester or polymethylglycidyl ester of bis(4'-carboxyphenyl)-1,4-benzenediimine; Formula II; Formula III, with the proviso that the monoglycidyl ester of Formula III may include the monoglycidyl ester of 4-carboxybiphenyl; Formula IV; or any combination of any two or more polyglycidyl esters or monoglycidyl ester compounds represented by the aforementioned Formulas I, II, III or IV; with

(B) one or more polyepoxides which are substantially free of mesogenic or rodlike moieties.

**11.** A blend of Claim 10 wherein component (B) is a diglycidyl ether of bisphenol A, a diglycidyl ether of 4,4'-dihydroxydiphenylmethane, a diglycidyl ether of 4,4'-dihydroxybenzophenone , a diglycidyl ether of 4,4'-dihydroxythiodiphenol, a diglycidyl ether of 1,1-bis(4-hydroxylphenyl)-1-phenylethane (bisphenol AP), or any combination thereof.

**12.** A blend comprising

(A) an advanced polyglycidyl ester composition prepared by reacting

(1) one or more polyglycidyl ester containing one or more mesogenic moieties, said polyglycidyl ester being those represented by either the following Formula I with the proviso that the polyglycidyl ester of Formula I may include the polyglycidyl ester of 4,4'-dicarboxystilbene or the polyglycidyl or polymethylglycidyl ester of bis(4'-carboxyphenyl)-1,4-benzenediimine; or Formula II;

or any combination of any two or more epoxy resins represented by the aforementioned Formulas I and II; with

(2) at least one compound having an average of more than one active hydrogen atom per molecule; and

wherein components (1) and (2) are employed in quantities which provide a ratio of active hydrogen atoms per epoxide group of from 0.01:1 to 0.95:1; and

(B) one or more polyepoxides which are substantially free of mesogenic or rodlike moieties.

**13.** A blend of Claim 12 wherein

(a) component (B) is hydroquinone, bisphenol A, 4,4'-dihydroxydiphenylmethane, 4,4'-thiodiphenol, 4,4'-sulfonyldiphenol, 4,4'-dihydroxydiphenyl oxide, 4,4'-dihydroxybenzophenone, 1,1-bis(4-hydroxyphenyl)-1-phenylethane, 3,3',5,5'-tetrachlorobisphenol A, 3,3'-dimethoxybisphenol A, 4,4'-dihydroxybiphenyl, 4,4'-dihydroxy-α,α'-diethylstilbene, 4,4'-dihydroxy-α-methylstilbene, 4,4'-dihydroxybenzanilide, 4,4'-dihydroxy-2,2'-dimethylazoxybenzene, 4,4'-dihydroxy-α-cyanostilbene, bis(4-hydroxyphenyl)terephthalate, N,N'-bis(4-hydroxyphenyl)terephthalamide, bis(4'-hydroxybiphenyl)-terephthalate, 4,4'-dihydroxyphenylbenzoate, bis(4'-hydroxyphenyl)-1,4-benzenediimine, 4,4''-dihydroxybiphenylbenzoate, 1,4-bis(4'-hydroxyphenyl-1'-carboxamide)benzene, 1,4-bis(4'-hydroxyphenyl-1'-carboxy)benzene, 4,4'-bis(4''-hydroxyphenyl-1''-carboxy)biphenyl, terephthalic acid, 4,4'-benzanilide dicarboxylic acid, 4,4'-phenylbenzoate dicarboxylic acid, 4,4'-stilbenedicarboxylic acid, 4,4'-dicarboxybiphenyl, 4,4'-dicarboxydiphenylazomethine, aniline, 4'-sulfonamido-N-phenylbenzamide, 4'-sulfonamido-N'-phenyl-4-chlorobenzamide, 4-amino-1-phenylbenzoate, 4-amino-N-phenylbenzamide, N-phenyl-4-aminophenyl-1-carboxamide, phenyl-4-aminobenzoate, biphenyl-4-aminobenzoate, 1-phenyl-4'-aminophenylterephthalate and mixtures thereof; and

(b) components (1) and (2) are employed in amounts which provide a ratio of active hydrogen atoms per epoxide group of from 0.1:1 to 0.5:1.;

(c) component (A) is present in an amount of from 10 to 50 percent by weight based on the combined quantities of components (A) and (B); and

(d) component (B) is present in an amount of from 90 to 50 percent by weight based on the combined quantities of components (A) and (B).

**14.** A blend comprising
(A) one or more polyglycidyl esters containing an average of more than one vicinal epoxide group per molecule and one or more mesogenic moieties per molecule; and
(B) one or more monoglycidyl ester compounds containing only one vicinal epoxide group per molecule and one or more mesogenic moieties per molecule.

**15.** A blend of Claim 14 wherein component (A) is present in an amount of from about 10 to about 50 percent by weight based upon the combined weight of components (A) and (B) and component (B) is present in an amount of from about 90 to about 50 percent by weight based upon the combined weight of components (A) and (B).

**16.** A curable composition comprising at least one polyglycidyl ester of Claim 1, 2, or 3 and a curing amount of a suitable curing agent therefor.

**17.** A curable composition comprising at least one advanced polyglycidyl ester of Claim 4, 5,6, or 7 and a curing amount of a suitable curing agent therefor.

**18.** A curable composition comprising at least one blend of Claim 10, 11,12,13, 14, or 15 and a curing amount of a suitable curing agent therefor.

**19.** The product resulting from curing the curable composition of Claim 16.

**20.** The product resulting from orienting a curable composition of Claim 16 either prior to curing, during curing or both prior to or during curing.

**21.** The product of Claim 20 wherein said orientation is accomplished by means of an electric or magnetic field, or by the application of drawing or shear forces.

**22.** The product resulting from curing the curable composition of Claim 17.

**23.** The product resulting from orienting a curable composition of Claim 17 either prior to curing, during curing or both prior to or during curing.

**24.** The product of Claim 23 wherein said orientation is accomplished by means of an electric or magnetic field, or by the application of drawing or shear forces.

**25.** The product resulting from curing the curable composition of Claim 18.

**26.** The product resulting from orienting a curable composition of Claim 18 either prior to curing, during curing or both prior to or during curing.

**27.** The product of Claim 26 wherein said orientation is accomplished by means of an electric or magnetic field, or by the application of drawing or shear forces.

**28.** A phenoxy type resin prepared by reacting
(A) one or more polyglycidyl esters containing one or more mesogenic moieties, said polyglycidyl esters being those represented by either Formula I with the proviso that the polyglycidyl esters of Formula I may include the polyglycidyl ester of 4,4'-dicarboxystilbene or the polyglycidyl ester of bis(4'-carboxyphenyl)-1,4-benzenedimine; or Formula II; with
(B) at least one compound having an average of more than one active hydrogen atom per molecule;
wherein components (A) and (B) are employed in quantities which provide a ratio of active hydrogen atoms per epoxide group of from about 0.96:1 to about 1.05:1.